# EUROPEAN PATENT APPLICATION

(11) **EP 4 763 103 A1**
(43) Date of publication of application: **24.06.2026**
(21) Application number: 24859737.9
(22) Date of filing: 27.08.2024
(51) Int. Cl.: A61B 17/135

(54) **HEMOSTASIS INSTRUMENT**

(30) Priority: 28.08.2023 JP 2023137824
(71) Applicant: Terumo Kabushiki Kaisha, Tokyo 151-0072 (JP)
(72) Inventor: HAYASHI, Koki, Fujinomiya-shi, Shizuoka 418-0015 (JP); TSURUMAKI, Shunto, Fujinomiya-shi, Shizuoka 418-0015 (JP)
(74) Representative: Casalonga
(86) International application number: PCT/JP2024/030396
(87) International publication number: WO 2025/047705

(57) **Abstract**

The purpose of the present invention is to provide a hemostatic device capable of preventing a proximal portion of a cover member from digging into a body surface of a patient who wears the hemostatic device and also capable of preventing an article or the like from being caught on the cover member in a state in which the hemostatic device is worn on the patient while preventing a support member from falling from the cover member. For achieving the purpose, a hemostatic device (10) includes a cover member (100) having a main body (110), wherein the main body (110) has a two-layer structure including a holding portion (130) that holds the support member (300), the holding portion has an upper surface region (131) located on an upper surface (300b) side of the support member, a lower surface region (132) located on a lower surface side of the support member and facing the upper surface region across the support member, and a curved region (133) connecting the upper surface region and the lower surface region on a proximal side of the support member, the curved region is formed by folding back a part of the main body toward a first band body, an edge (121) of the lower surface region has a fixed region (150A, 150B) fixed to the upper surface region, and the fixed region is positioned on a distal side with respect to the support member and located at a position surrounding edge portions (306c, 306d) of the support member in plan view.

## Description

### Technical Field

The present invention relates to a hemostatic device.

### Background Art

As one of catheter procedures, there is known a procedure in which various medical elongated bodies are introduced into a blood vessel of a limb such as an arm or hand of a patient through a puncture site formed by puncturing the blood vessel to perform a diagnosis or a therapy at a lesion site. For example, Patent Literature 1 discloses a hemostatic device for stopping bleeding at a puncture site formed to enable access to a blood vessel (including a distal radial artery) running in a hand.

The hemostatic device disclosed in Patent Literature 1 includes a pressing member that applies a compressive force to the puncture site formed in a hand of the patient (hereinafter, also simply referred to as "puncture site"), and a cover member configured to cover the pressing member. Furthermore, the cover member includes a plurality of band bodies for securing the pressing member to the hand of the patient, and a support member that presses the pressing member against the hand of the patient. The pressing member and the support member are disposed at predetermined positions of the cover member.

### Citation List

### Patent Literature

Patent Literature 1: US 2019/0133602 A

### Summary of Invention

### Technical Problem

When stopping bleeding at the puncture site formed in the limb of the patient, an operator such as a doctor (hereinafter, referred to as "operator") places the hemostatic device on the limb of the patient so as to cover the puncture site with the cover member. When the operator places the hemostatic device so as to cover the puncture site with the cover member, the pressing member and the support member located at the cover member are placed at the puncture site. The hemostatic device can stop bleeding at the puncture site as the pressing member applies a compressive force to the puncture site in a state where the pressing member and the support member are placed at the puncture site.

When hemostasis is performed using the above hemostatic device, the following problems may occur.

If the patient wearing the hemostatic device moves his/her limb during hemostasis using the pressing member, an edge of the cover member may dig into a body surface of the limb of the patient. In particular, in a case where the limb on which the puncture site is formed is a hand, a proximal portion of the cover member (an end placed on a forearm side) digs into the body surface near the wrist if the patient wearing the hemostatic device moves to twist the wrist toward a dorsal side of his/her hand, for example. Therefore, the hemostatic device may give the patient discomfort such as pain caused by the proximal portion of the cover member digging into the body surface of the limb.

In order to solve the above problem, for example, it is conceivable to take measures to prevent the proximal portion of the cover member from digging into the body surface near the wrist by making the proximal portion of the cover member flexible. However, in the hemostatic device disclosed in Patent Literature 1, the support member that presses the pressing member against the hand of the patient is disposed over a region including the vicinity of the proximal portion of the cover member. Therefore, in the hemostatic device disclosed in Patent Literature 1, in order to prevent the support member from falling off from the cover member, the support member is fixed to the cover member in a wide range including the vicinity of the proximal portion of the cover member. Accordingly, in the hemostatic device disclosed in Patent Literature 1, it is difficult to adopt a structure that enhances the flexibility in the vicinity of the proximal portion of the cover member. Therefore, the hemostatic device disclosed in Patent Literature 1 has difficulty in addressing the problem that the proximal portion of the cover member digs into the body surface of the limb.

The present invention has been made based on the above problems, and an object thereof is to provide a hemostatic device capable of preventing a proximal portion of a cover member from digging into a body surface of a patient wearing the hemostatic device, and capable of preventing an article or the like from being caught on the cover member in a state where the hemostatic device is worn on the patient, while preventing a support member from falling off from the cover member.

### Solution to Problem

The present invention is achieved by any one of the following aspects (1) to (9).
(1) A hemostatic device comprising: a cover member configured to cover a puncture site formed in a patient; a pressing member disposed on the cover member and configured to compress the puncture site; and a support member disposed on the cover member so as to cover the pressing member, wherein the cover member includes a main body where the pressing member is located, a first band body configured to be placed between fingers of the patient and extending in a first direction from the main body, a second band body extending in a second direction different from the first direction from the main body, and a third band body facing the second band body across the pressing member and extending from the main body in a third direction different from the first direction and the second direction, the main body has a two-layer structure including a holding portion that holds the support member, the holding portion has an upper surface region located on an upper surface side of the support member, a lower surface region located on a lower surface side of the support member and facing the upper surface region across the support member, and a curved region connecting the upper surface region and the lower surface region on a proximal side of the support member, the curved region is formed by folding back a part of the main body toward the first band body, the support member includes an upper end portion located on a side of the first band body and serves as an end of the support member, a lower end portion serving as an end opposite to the upper end portion, an intermediate portion positioned between the upper end portion and the lower end portion, and an edge portion connecting the upper end portion and the intermediate portion, an edge of the lower surface region has a fixed region fixed to the upper surface region, and the fixed region is positioned on a distal side with respect to the support member and located at a position surrounding the edge portion in plan view.
(2) The hemostatic device according to (1), wherein the fixed region includes a first fixed region located on a distal side with respect to the upper end portion of the support member and a second fixed region provided continuously with the first fixed region and extending from the first fixed region toward the lower end portion of the support member, the holding portion has a clearance between the support member and the first fixed region in plan view, and a width of the clearance is equal to or larger than a thickness of the upper end portion of the support member.
(3) The hemostatic device according to (2), wherein the second band body and the third band body extend from the main body in the second direction and the third direction via the second fixed region located between the second and third band bodies and the main body, and the second band body and the third band body have deflected portions located on a side of the first band body with respect to an end of the second fixed region located on a side of the curved region in plan view.
(4) The hemostatic device according to (2) or (3), wherein the pressing member is an inflatable member inflatable by injection of a fluid, the inflatable member is connected to an injection member for injecting the fluid into the inflatable member via a tube member, the first band body has a slit extending in a longitudinal direction of the first band body, the first fixed region includes a left fixed region and a right fixed region facing the left fixed region across an extension of the slit, and the tube member is located between the left fixed region and the right fixed region in plan view, passes through a non-fixed region in which the edge of the lower surface region is not fixed to the upper surface region, and is inserted from an inner surface side of the first band body toward an outer surface side of the first band body through the slit.
(5) The hemostatic device according to (4), wherein the inflatable member includes a sheet material connected to the cover member in such a manner that an inner cavity into which the fluid is capable of being injected is defined with the lower surface region, and an edge located on a side of the lower end portion of the support member in the sheet material is connected to the curved region.
(6) The hemostatic device according to any one of (1) to (5), wherein the lower surface region includes a constricted portion configured in such a manner that a width of the lower surface region decreases from a side of the first band body toward the curved region, and the constricted portion is not fixed to the upper surface region.
(7) The hemostatic device according to any one of (2), (3), (4), and (5), wherein a maximum width of the support member is larger than a maximum width of the holding portion between the curved region and the end of the second fixed region on a side of the curved region.
(8) The hemostatic device according to any one of (1) to (7), wherein an end of the fixed region on a side of the curved region is located on a side of the first band body with respect to the intermediate portion of the support member.
(9) The hemostatic device according to any one of (2), (3), (4), (5), and (7), wherein the first band body has a first end located on a side of the main body of the cover member, and the first end of the first band body is connected to an inner surface side of the main body in a state of being spaced apart from the first fixed region.

### Advantageous Effects of Invention

In the hemostatic device described above, the main body included in the cover member includes the holding portion having a two-layer structure to hold the support member. The holding portion has the curved region formed by folding back a part of the main body toward the first band body. In the hemostatic device, the curved region of the holding portion comes into contact with the body surface of the patient when the patient moves his/her limb in a state where the hemostatic device is worn on the limb of the patient. Since the curved region is formed in a curved shape, the hemostatic device can prevent a proximal portion of the cover member from digging into the body surface of the patient. In addition, in the hemostatic device, the edge of the lower surface region of the holding portion that holds the support member has the fixed region fixed to the upper surface region of the holding portion. The fixed region is located on a distal side with respect to the support member and is located at a position surrounding the edge portion that connects the upper end portion and the intermediate portion of the support member in plan view. Therefore, in the hemostatic device, the support member can be prevented from slipping out from the lower end portion side to the outside of the holding portion by the curved region formed by folding back a part of the main body, and the support member can be prevented from slipping out from the upper end portion side to the outside of the holding portion by the fixed region located at the upper end portion of the support member. In addition, the fixed region is located on the distal side with respect to the support member and is located at a position surrounding the edge portion that connects the upper end portion and the intermediate portion of the support member in plan view. Therefore, in a state where the hemostatic device is worn on the limb of the patient, the cover member can suitably prevent the edge portion of the support member from being lifted via the upper surface region by the fixed region located at a position surrounding the edge portion of the support member. Accordingly, by preventing the edge portion located on the distal side of the support member from being lifted, the hemostatic device can prevent a situation in which an article or the like is likely to be caught on the edge portion (cover member that covers the edge portion located on the distal side of the support member) located on the distal side of the support member. Thus, according to the hemostatic device described above, it is possible to prevent the proximal portion of the cover member from digging into the body surface of the patient wearing the hemostatic device, and to prevent an article or the like from being caught on the cover member in a state where the hemostatic device is worn on the patient, while preventing the support member from falling off from the cover member.

### Brief Description of Drawings

Fig. 1 is a plan view illustrating a hemostatic device according to an embodiment as viewed from an outer surface side of each band body.
Fig. 2 is a plan view illustrating the hemostatic device according to the embodiment as viewed from an inner surface side of each band body.
Fig. 3 is an enlarged view illustrating a part of the hemostatic device as viewed from an outer surface side of a main body of a cover member.
Fig. 4 is an enlarged view illustrating a part of the hemostatic device as viewed from an inner surface side of the main body of the cover member.
Fig. 5 is an enlarged view illustrating a part of the hemostatic device as viewed from the outer surface side of the main body of the cover member.
Fig. 6 is a partial cross-sectional view of the hemostatic device taken along a line 6A-6A illustrated in Fig. 5, and illustrates a state when an inflatable portion is inflated.
Fig. 7 is a partial cross-sectional view of the hemostatic device taken along a line 7A-7A illustrated in Fig. 5, and illustrates a state when the inflatable portion is inflated.
Fig. 8 is an enlarged perspective view illustrating a part of the hemostatic device.
Fig. 9 is a plan view illustrating a positional relation among a support member, a pressing member, and a holding portion.
Fig. 10 is a perspective view of the support member as viewed from a lower end portion.
Fig. 11 is a perspective view of the support member as viewed from an upper end portion.
Fig. 12 is a perspective view illustrating the lower surface side of the support member.
Fig. 13 is a perspective view illustrating the lower surface side of the support member.
Fig. 14 is a diagram illustrating a hand (right hand) of a patient for which the hemostatic device is to be used.
Fig. 15 is a diagram schematically illustrating a usage example of the hemostatic device.
Fig. 16 is a diagram schematically illustrating the usage example of the hemostatic device.
Fig. 17 is a diagram schematically illustrating the usage example of the hemostatic device.
Fig. 18 is a partial cross-sectional view taken along a line 18A-18A illustrated in Fig. 17.
Fig. 19 is a partial cross-sectional view taken along a line 19A-19A illustrated in Fig. 17.
Fig. 20 is a diagram schematically illustrating a state as viewed in a direction of an arrow 20A illustrated in Fig. 17.
Fig. 21 is a diagram illustrating a usage example of a hemostatic device according to a comparative example.
Fig. 22 is a plan view illustrating a fixed region according to Comparative Example 1.
Fig. 23 is a plan view illustrating a fixed region according to Comparative Example 2.
Fig. 24 is a plan view illustrating a fixed region and a non-fixed region according to a modification.
Fig. 25 is a plan view illustrating a fixed region and a non-fixed region according to a modification.

### Description of Embodiments

An embodiment of the present invention will be described below with reference to the accompanying drawings. The following description does not limit the technical scope or the significance of each term set forth in the claims. The dimensional ratios in the drawings are exaggerated for convenience of description and may be different from actual ratios.

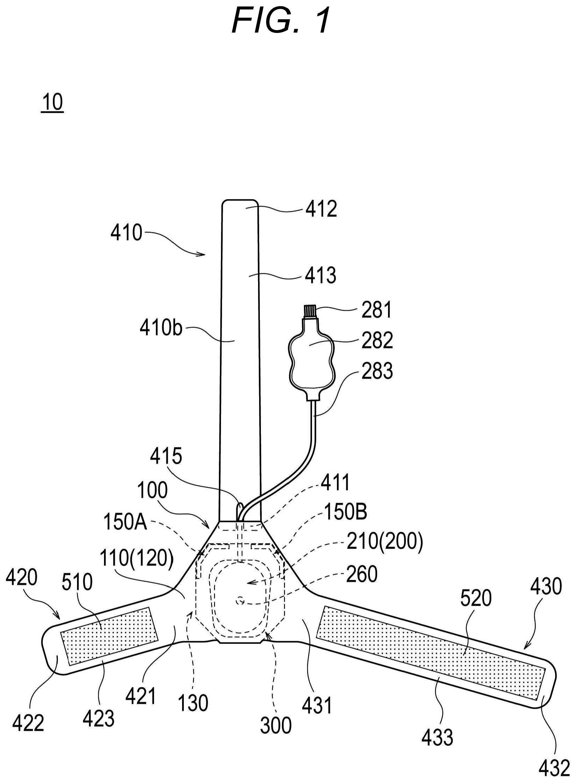

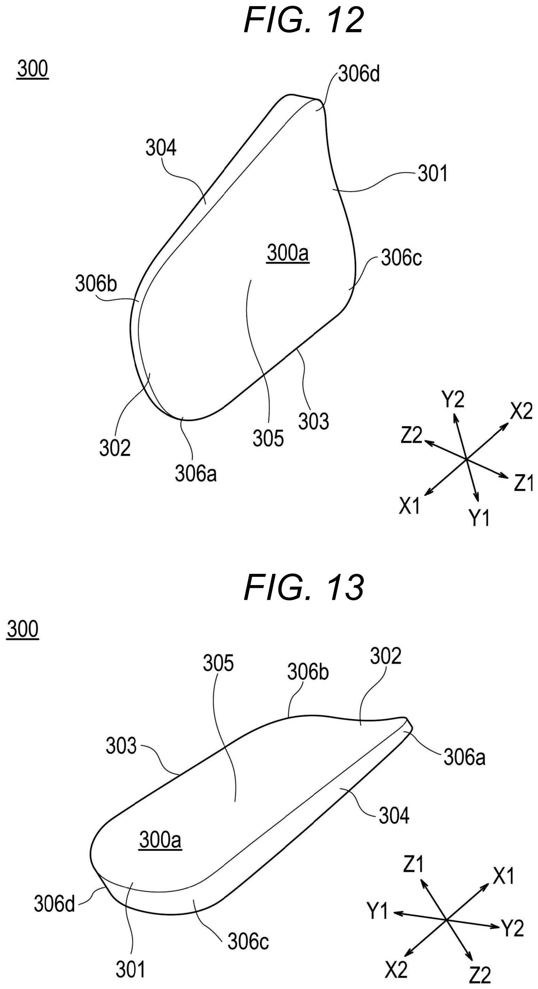

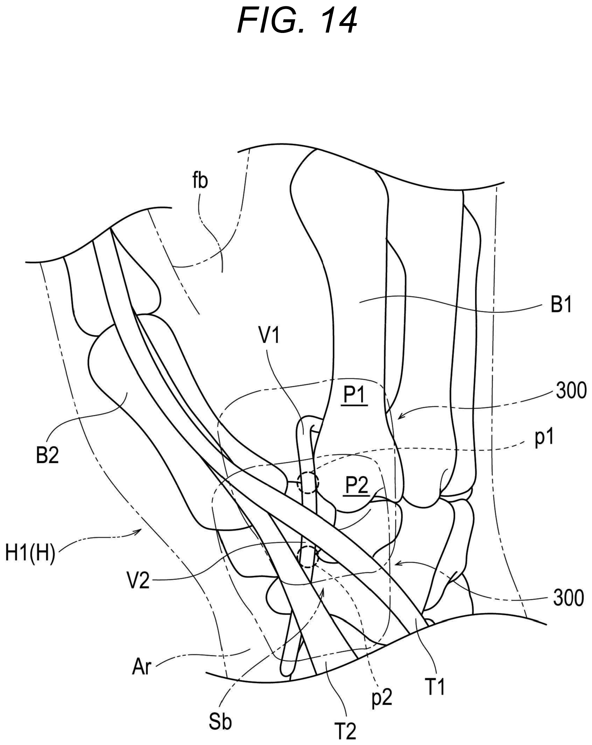

Figs. 1 to 13 are diagrams for describing a hemostatic device 10 according to an embodiment. Figs. 14 to 19 are diagrams for describing usage examples of the hemostatic device 10 according to the embodiment.

As illustrated in Figs. 14, 17, 18, and 19, for example, the hemostatic device 10 can be used to stop bleeding at a first puncture site p1 formed in a hand H located on a distal side (fingertip side) with respect to a forearm Ar of a patient when a sheath tube 610 of an introducer 600 placed at the first puncture site p1 is removed.

The specific position of the puncture site where bleeding is to be stopped by the hemostatic device 10 is not particularly limited, but in the present specification, the first puncture site p1 and a second puncture site p2 are described as an example of the puncture site.

As illustrated in Figs. 14, 17, 18, and 19, the first puncture site p1 is a puncture site formed in a distal radial artery (hereinafter, also referred to as "blood vessel V1") located on a distal side with respect to a snuff box Sb of a palmar artery running on the dorsal side of a right hand H1 located on the distal side with respect to the forearm Ar of the patient. In addition, the first puncture site p1 is located at a predetermined position, which is a position between a metacarpal bone B1 of an index finger and a metacarpal bone B2 of a thumb and avoiding the metacarpal bone B1 of the index finger and the metacarpal bone B2 of the thumb. Note that the snuff box Sb is a cavity in the hand located in the vicinity of the radius when the patient spreads the thumb of the hand H.

As illustrated in Fig. 14, the second puncture site p2 is a puncture site formed in an artery V2 located in the snuff box Sb of the palmar artery running on the dorsal side of the right hand H1. The second puncture site p2 is located on the forearm Ar side (proximal side) of the patient with respect to the first puncture site p1, and is located between an extensor pollicis longus muscle tendon T1 and an extensor pollicis brevis muscle tendon T2 located at the dorsal side of the right hand H1 of the patient. In addition, the second puncture site p2 is located at a predetermined position avoiding the position of the metacarpal bone B1 of the index finger and the position of the metacarpal bone B2 of the thumb.

Note that the hemostatic device 10 can also be applied to hemostasis of a puncture site on the left hand of the patient formed at a position corresponding to the first puncture site p1 on the right hand H1 of the patient or a puncture site on the left hand of the patient formed at a position corresponding to the second puncture site p2 on the right hand H1 of the patient.

Hereinafter, the hemostatic device 10 will be described in detail.

### <Hemostatic Device 10>

As schematically illustrated in Figs. 1, 2, 15, 16, 17, 18, and 19, the hemostatic device 10 includes a cover member 100 configured to cover the first puncture site p1, a pressing member 200 connected to the cover member 100 and configured to compress the first puncture site p1, and a support member 300 disposed on the cover member 100 so as to cover the pressing member 200.

In the present specification, the fingertip side of a finger is defined as a "distal side", and the forearm Ar side is defined as a "proximal side" in a state where the hemostatic device 10 is worn on the right hand H1 of the patient. A "distal side" and a "proximal side" used in the description of the hemostatic device 10 are also defined similarly to directions defined above.

### <Pressing Member 200>

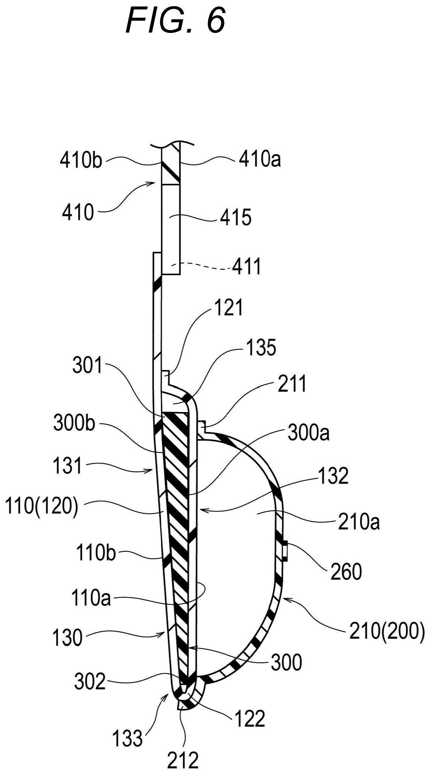

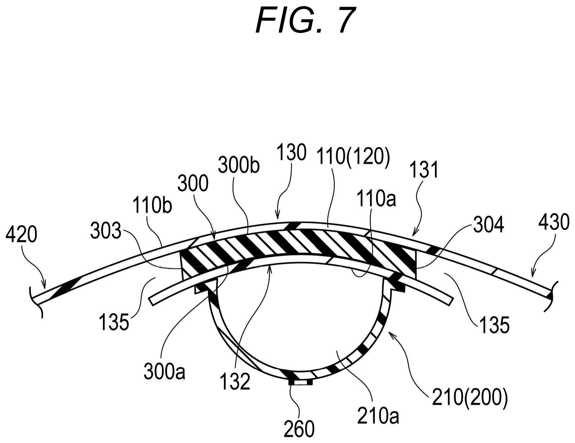

The pressing member 200 can be constituted by, for example, an inflatable member 210 that can be inflated by injection of a fluid as illustrated in Figs. 6 and 7. In the present embodiment, an example in which the pressing member 200 is constituted by the inflatable member 210 will be described in the following description.

The inflatable member 210 includes an inner cavity 210a into which the fluid can be injected. The inflatable member 210 is configured to be inflated as the fluid is injected into the inner cavity 210a and deflated as the fluid injected into the inner cavity 210a is discharged. The fluid used for the inflation of the inflatable member 210 is, for example, a gas such as air. Figs. 6 and 7 illustrate cross-sectional views of the inflatable member 210 in an inflated state. Note that, in the cross-sectional views of Figs. 6, 7, 18, and 19, a tube member 283 is not illustrated.

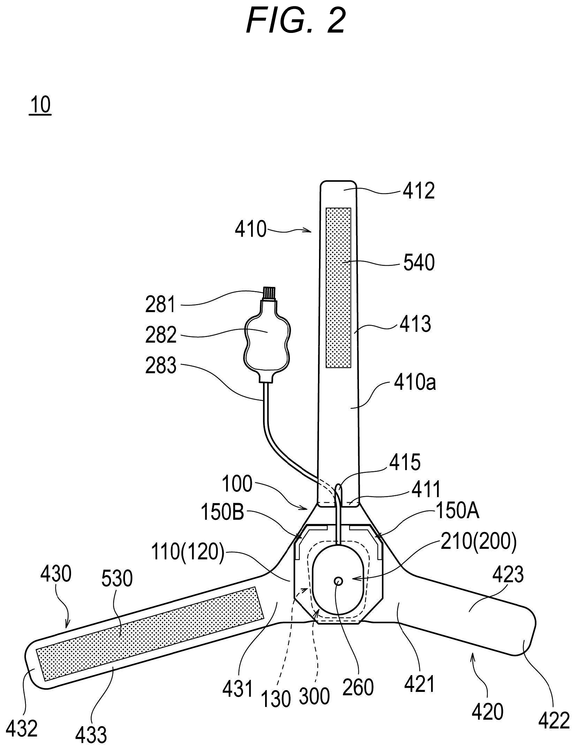

The tube member 283 (see Figs. 1 and 2) to be described later is connected to the inner cavity 210a of the inflatable member 210.

As illustrated in Figs. 6 and 7, the inflatable member 210 can include, for example, a film-shaped member (sheet material) connected to a sheet material 120 that constitutes a main body 110 of the cover member 100.

The sheet material constituting the inflatable member 210 can be connected to the cover member 100 such that the inner cavity 210a into which the fluid can be injected is defined with a lower surface region 132 of the holding portion 130 made of the sheet material 120 constituting the main body 110 of the cover member 100.

An outer peripheral edge of the film-shaped member forming the inflatable member 210 defines an edge of the inflatable member 210. The edge of the inflatable member 210 can be connected to the sheet material 120 by, for example, fusing, using an adhesive, or the like.

As illustrated in Fig. 6, an edge 212 located at a lower end portion 302 of the support member 300 in the sheet material constituting the inflatable member 210 can be connected to a curved region 133 formed by the cover member 100. As illustrated in Fig. 6, an edge 211 located at an upper end portion 301 side of the support member 300 in the sheet material constituting the inflatable member 210 can be fixed to the sheet material 120 at a predetermined position overlapping a lower surface 300a of the support member 300 in the plane direction.

The film-shaped member constituting the inflatable member 210 can be made of, for example, a resin material having a predetermined thickness.

A material for the film-shaped member constituting the inflatable member 210 is not particularly limited, and examples thereof include polyvinyl chloride, ethylene-vinyl acetate copolymer (EVA), polyvinylidene chloride, silicone, polyamide (nylon), polyurethane, polyolefin such as polyethylene, polypropylene, or polybutadiene, polyester such as polyethylene terephthalate (PET) or polybutylene terephthalate (PBT), various thermoplastic elastomers such as polyamide elastomer (nylon elastomer), polyurethane elastomer, or polyester elastomer, or any combination thereof (e.g., blended resin, polymer alloy, laminate, or the like).

The inflatable member 210 is disposed on an inner surface 110a side of the main body 110 of the cover member 100 as illustrated in Figs. 6 and 7.

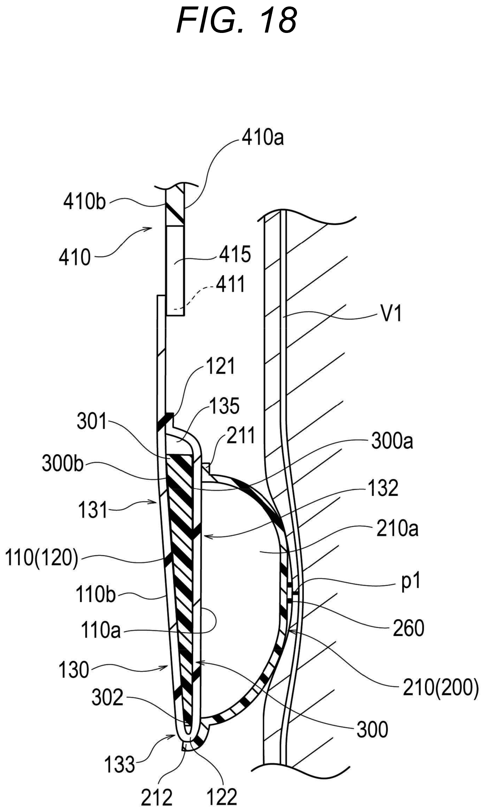

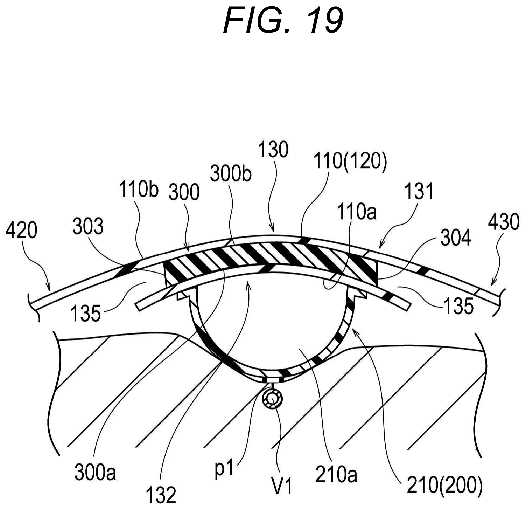

In the present embodiment, the inner surface 110a of the main body 110 is defined by a surface to be placed on a body surface side of the right hand H1 in the lower surface region 132 of the holding portion 130 (see Figs. 18 and 19). An outer surface 110b of the main body 110 is defined by a surface on a side facing the outside in an upper surface region 131 of the holding portion 130.

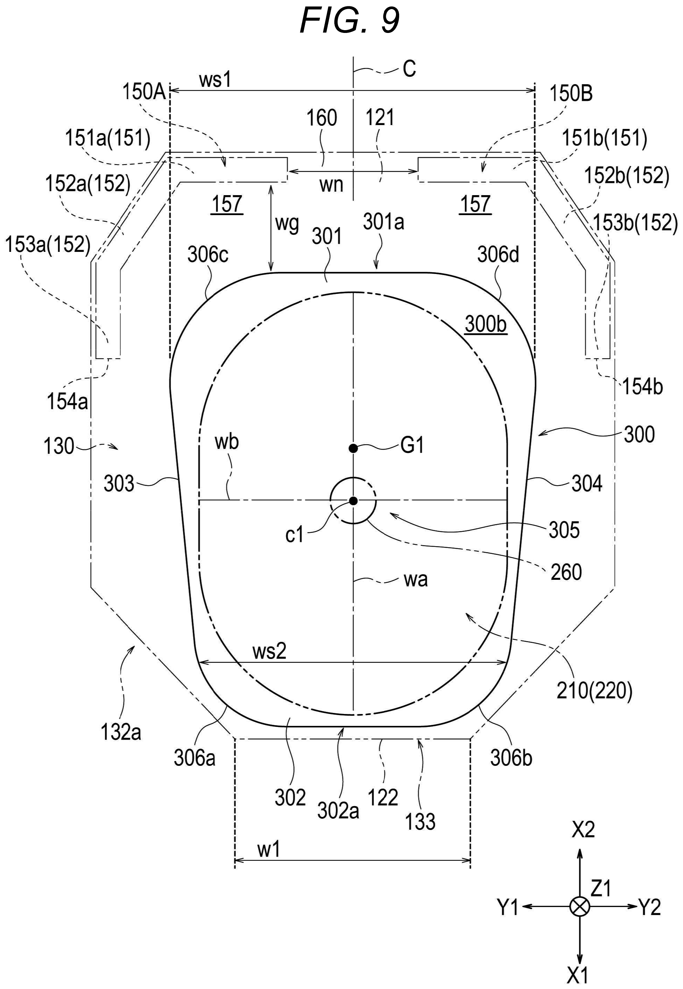

Fig. 9 is a plan view of the support member 300 and illustrates a positional relation among the inflatable member 210 in a deflated state, the support member 300, and the holding portion 130. Fig. 9 is a plan view as viewed from an upper surface 300b of the support member 300. The inflatable member 210 and the holding portion 130 are indicated by a dash-dot-dot line in Fig. 9.

The inflatable member 210 has a longitudinal width wa passing through a center c1 of the inflatable member 210 and a lateral width wb that is orthogonal to the longitudinal width wa and passes through the center c1 of the inflatable member 210 in a state where the inflatable member 210 is deflated. Note that the longitudinal width wa and the lateral width wb are dimensions that do not include the edge located on the outer periphery of the inflatable member 210, and indicate the longitudinal width and the lateral width in an outer shape of the inflatable member 210 illustrated in the plan view of Fig. 9.

The center c1 of the inflatable member 210 is located at a center of the outer shape illustrated in the plan view of Fig. 9. In the present embodiment, the center c1 is located at a position where the center position of an axis (long axis) along the longitudinal width wa and the center position of an axis (short axis) along the lateral width wb intersect. Note that, in the present embodiment, the center position of the axis along the longitudinal width wa is a line segment passing through a point where distances from both ends of the axis along the longitudinal width wa are equal on the axis along the longitudinal width wa. In addition, the center position of the axis along the lateral width wb is a line segment passing through a point where distances from both ends of the axis along the lateral width wb are equal on the axis along the lateral width wb.

The lateral width wb of the inflatable member 210 is shorter than the longitudinal width wa. In the present embodiment, the inflatable member 210 has a substantially oval shape that is rotationally symmetric with respect to the center c1 in the plan view illustrated in Fig. 9.

In the plan view illustrated in Fig. 9, the inflatable member 210 is disposed on the cover member 100 in a state where the center c1 of the inflatable member 210 is located on the lower end portion 302 side of the support member 300 with respect to a center of gravity G of an outer shape of the support member 300.

In the plan view illustrated in Fig. 9, the inflatable member 210 is connected to the lower surface region 132 such that a part (a part on a proximal portion side) of the inflatable member 210 is located in a constricted portion 132a of the holding portion 130 (see Fig. 6).

As described above, the edge 212 located at the lower end portion 302 of the support member 300 in the sheet material constituting the inflatable member 210 is connected to the curved region 133 formed by the cover member 100 (see Fig. 6). Therefore, the inflatable member 210 can be disposed such that the center c1 of the inflatable member 210 is located at a position shifted to the proximal portion 122 side of the main body 110 in the longitudinal width direction (length in the vertical direction in Fig. 9) of the main body 110.

Note that the planar shape of the inflatable member 210 in plan view is not particularly limited. For example, the planar shape of the inflatable member 210 may be a circle, an ellipse, or a rectangle. When the inflatable member 210 is configured to have a planar shape such as a circular shape, an elliptical shape, or a rectangular shape, the center of the inflatable member 210 can be defined by a center position of the outer shape in plan view.

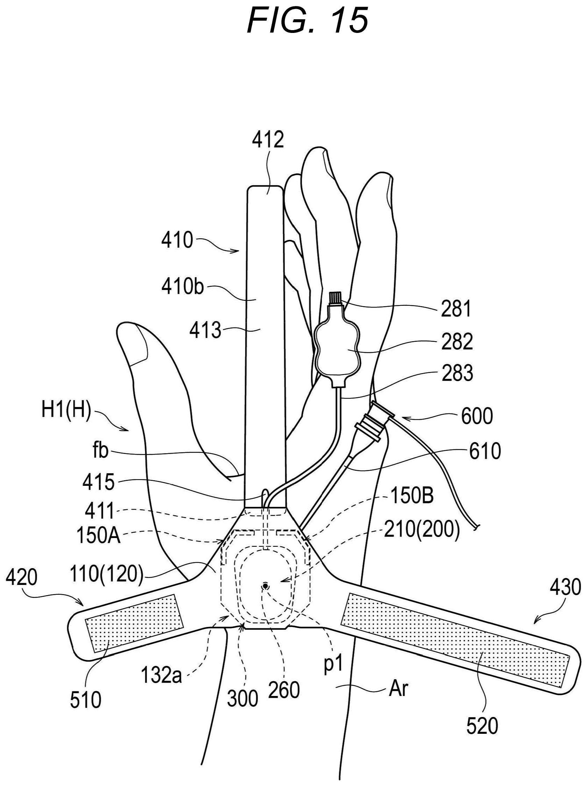

The inflatable member 210 includes a marker portion 260 for aligning the inflatable member 210 with the first puncture site p1 as illustrated in Figs. 15, 16, and 17.

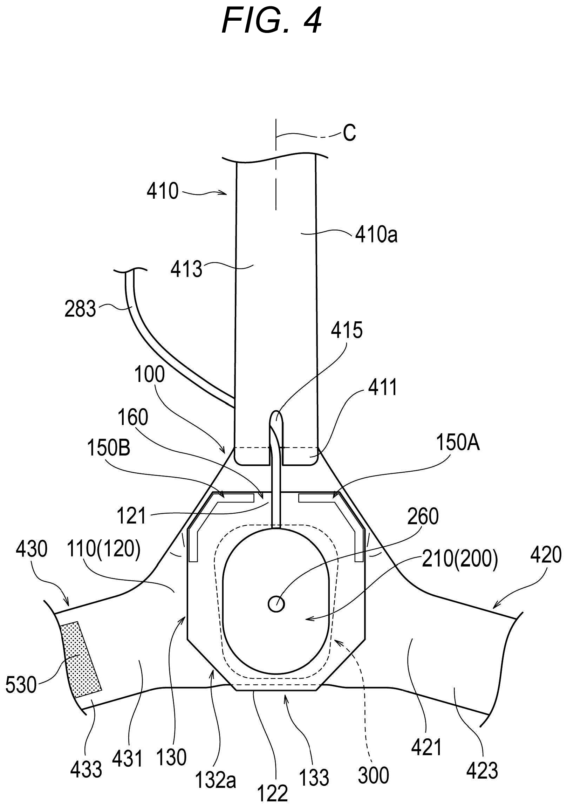

As illustrated in Figs. 4, 6, 7, and 9, the marker portion 260 can include a transparent central part surrounding the center c1 of the inflatable member 210 and a colored circular frame part surrounding the central part.

The marker portion 260 is provided on an outer surface of the inflatable member 210 as illustrated in Figs. 6 and 7. Note that the marker portion 260 may be provided on an inner surface of the inflatable member 210 facing the inner cavity 210a.

The specific shape and color, a position in the plane direction of the inflatable member 210, a formation method, and the like of the marker portion 260 are not particularly limited. The marker portion 260 can also be constituted by, for example, a circular marker that is entirely colored, a marker including a transparent central part and a rectangular frame part, a rectangular marker that is entirely colored, or the like.

### <Cover Member 100>

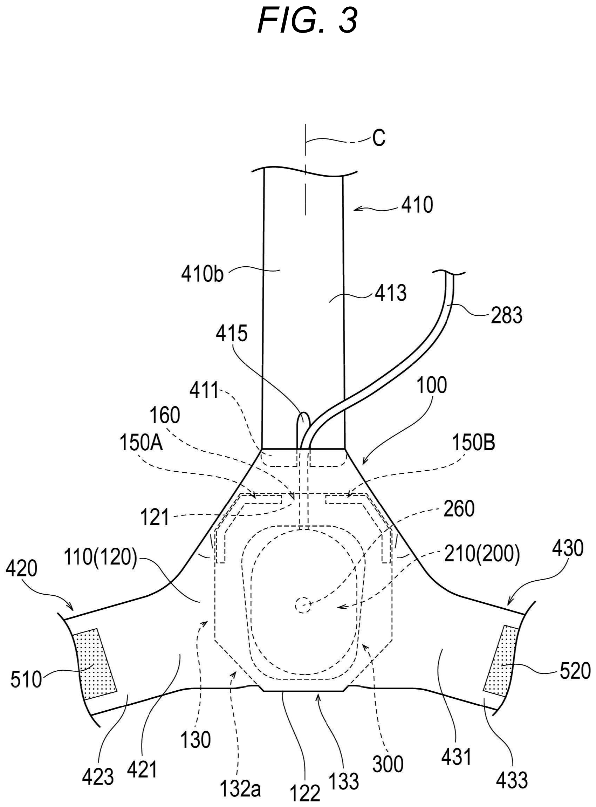

As illustrated in Figs. 3, 4, 5, and 17, the cover member 100 includes the main body 110 in which the inflatable member 210 is located, a first band body 410 that is configured to be placed between fingers of the patient and extends from the main body 110 in a first direction, a second band body 420 extending from the main body 110 in a second direction different from the first direction, and a third band body 430 facing the second band body 420 across the inflatable member 210 and extending from the main body 110 in a third direction different from the first direction and the second direction.

A region of the cover member 100 where the inflatable member 210 is disposed (a region overlapping the inflatable member 210 in the plan views illustrated in Figs. 3, 4, and 5) defines the main body 110.

As illustrated in Figs. 6 and 7, the main body 110 has a two-layer structure. The main body 110 having a two-layer structure includes the holding portion 130 that holds the support member 300.

As illustrated in Fig. 6, the holding portion 130 includes the upper surface region 131 located on the upper surface 300b side of the support member 300, the lower surface region 132 that is located on the lower surface 300a side of the support member 300 and faces the upper surface region 131 across the support member 300, and the curved region 133 connecting the upper surface region 131 and the lower surface region 132 on the proximal side of the support member 300 (the lower end portion 302 side of the support member 300).

The curved region 133 is formed by folding back a part of the sheet material 120 constituting the main body 110 toward the first band body 410. In the present embodiment, a part of the sheet material 120 constituting the main body 110 is folded back from the proximal side to the distal side of the main body 110. An insertion portion 135 in which the support member 300 can be disposed is formed between the upper surface region 131 and the lower surface region 132 of the sheet material 120 folded back in this manner. A section of the sheet material 120 where the insertion portion 135 is formed constitutes the holding portion 130 for holding the support member 300.

As illustrated in Fig. 6, the edge 121 (hereinafter, referred to as "edge 121 of the lower surface region 132") located on the distal side of the folded portion of the sheet material 120 constituting the main body 110 is fixed to the sheet material 120 by means of fixed regions 150A and 150B described later at a position on the distal side with respect to the upper end portion 301 of the support member 300.

The proximal portion 122 of the cover member 100, which is formed of a part of the sheet material 120 constituting the main body 110, is located in the curved region 133.

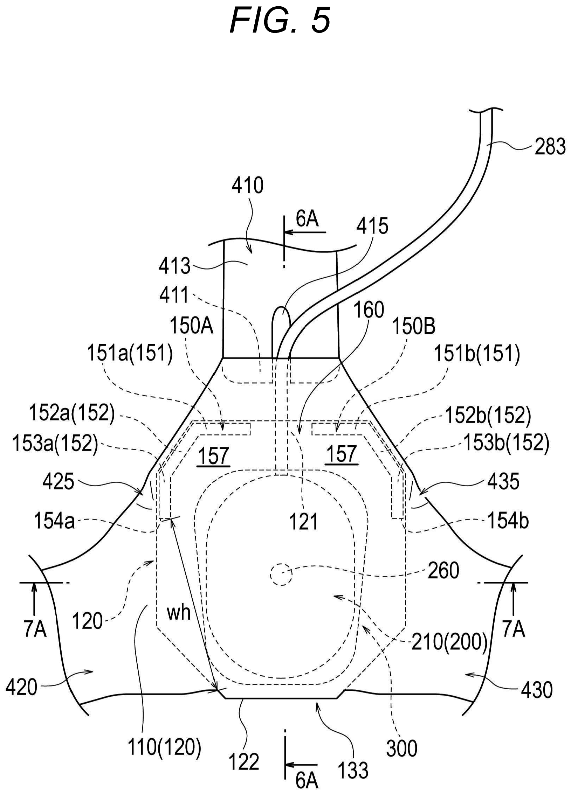

As illustrated in Figs. 5 and 6, the insertion portion 135 of the holding portion 130 is closed at a position on the upper end portion 301 side of the support member 300 except for a non-fixed region 160 to be described later.

As illustrated in Figs. 5 and 6, a section of the insertion portion 135 of the holding portion 130 on the lower end portion 302 side of the support member 300 is closed by the curved region 133.

In addition, as illustrated in Figs. 5 and 7, a section of the insertion portion 135 of the holding portion 130 located on the proximal portion 122 side of the lower surface region 132 with respect to ends 154a and 154b of the fixed regions 150A and 150B to be described later communicates with the outside.

As illustrated in Figs. 5 and 9, the lower surface region 132 has the constricted portion 132a located on the curved region 133 side.

As illustrated in Figs. 5 and 9, in the constricted portion 132a, a width (lateral width) of the lower surface region 132 decreases from the first band body 410 side to the curved region 133 side. That is, the constricted portion 132a is configured such that the width of the lower surface region 132 gradually decreases from the distal side to the proximal side in the plan views illustrated in Figs. 5 and 9.

The constricted portion 132a can be configured such that, for example, the width decreases toward the curved region 133 side at substantially the same rate in a bilaterally symmetric manner with respect to a center line C of the first band body 410 as illustrated in Fig. 9.

As illustrated in Fig. 9, a width w1 of the curved region 133 is smaller than a width ws2 of the proximal portion (lower end portion 302) of the support member 300 in plan view.

In the present specification, the width ws2 of the proximal portion (lower end portion 302) of the support member 300 can be defined by a length of a straight line connecting a boundary between a side surface portion 303 and a first edge portion 306a and a boundary between a side surface portion 304 and a second edge portion 306b. In a case where the first edge portion 306a is formed in a curved shape as in the present embodiment, the boundary between the side surface portion 303 and the first edge portion 306a is a position where a tangent line of the side surface portion 303 starts to change in a curved manner. Similarly, in a case where the second edge portion 306b is formed in a curved shape as in the present embodiment, the boundary between the side surface portion 304 and the second edge portion 306b is a position where a tangent line of the side surface portion 304 starts to change in a curved manner. Note that, in a case where the edge portions 306a and 306b are not formed in a curved shape, the width ws2 of the proximal portion of the support member 300 can be defined by a length of a straight line connecting vertices of the edge portions 306a and 306b.

In the present embodiment, the constricted portion 132a is constituted by a non-fixed region. That is, the section constituting the constricted portion 132a in the lower surface region 132 is not fixed to the upper surface region 131.

The constricted portion 132a is located between the second band body 420 and the third band body 430 in the main body 110 as illustrated in Fig. 3. That is, if the second band body 420 is extended toward the third band body 430 or if the third band body 430 is extended toward the second band body 420 in the plan view illustrated in Fig. 3, the constricted portion 132a can be disposed in a region of the main body 110 where the second band body 420 and the third band body 430 overlap each other.

### <First Band Body 410, Second Band Body 420, Third Band Body 430>

The first band body 410 is connected to the main body 110 at a position facing the curved region 133 across the support member 300 as illustrated in Figs. 3 and 4.

The second band body 420 and the third band body 430 are integrally formed with the main body 110.

Note that, similar to the first band body 410, the second band body 420 and the third band body 430 may be formed of a member different from the main body 110 and connected to the main body 110. In addition, the first band body 410 may be formed integrally with the main body 110 similarly to the second band body 420 and the third band body 430.

As illustrated in Figs. 1, 2, 3, and 4, the first band body 410 includes a first end 411 connected to the main body 110 of the cover member 100, a second end 412 located opposite to the first end 411, and an intermediate portion 413 extending between the first end 411 and the second end 412.

As illustrated in Fig. 6, the first end 411 of the first band body 410 is connected to the main body 110 of the cover member 100. The first end 411 of the first band body 410 can be connected to the main body 110 by, for example, fusion or adhesion.

In the present specification, a direction (vertical direction in Fig. 5) parallel to the direction in which the intermediate portion 413 of the first band body 410 extends is defined as a "longitudinal direction of the first band body 410". In addition, a direction (lateral direction in Fig. 5) orthogonal to the longitudinal direction of the first band body 410 is defined as a "width direction of the first band body 410". The center line C (see Fig. 9) of the first band body 410 is defined by a straight line passing through a substantially central position in the width direction of the first band body 410 and extending in the longitudinal direction of the first band body 410.

As illustrated in Figs. 3 and 4, the first band body 410 has a slit 415 extending in the longitudinal direction of the first band body 410.

As illustrated in Fig. 6, the slit 415 penetrates an inner surface 410a and an outer surface 410b of the first band body 410.

As illustrated in Figs. 3 and 4, the tube member 283 included in an injection member 281 passes between a left fixed region 151a and a right fixed region 151b to be described later, and is inserted through the slit 415 from the inner surface 410a side of the first band body 410 toward the outer surface 410b side of the first band body 410. Therefore, as illustrated in Figs. 1 and 2, the injection member 281 and a buffer member 282 which are connected to the tube member 283 are located on the outer surface 410b side of the first band body 410 together with a part of the tube member 283.

The slit 415 can be configured to extend, for example, by a predetermined length from the first end 411 of the first band body 410 along the longitudinal direction of the first band body 410.

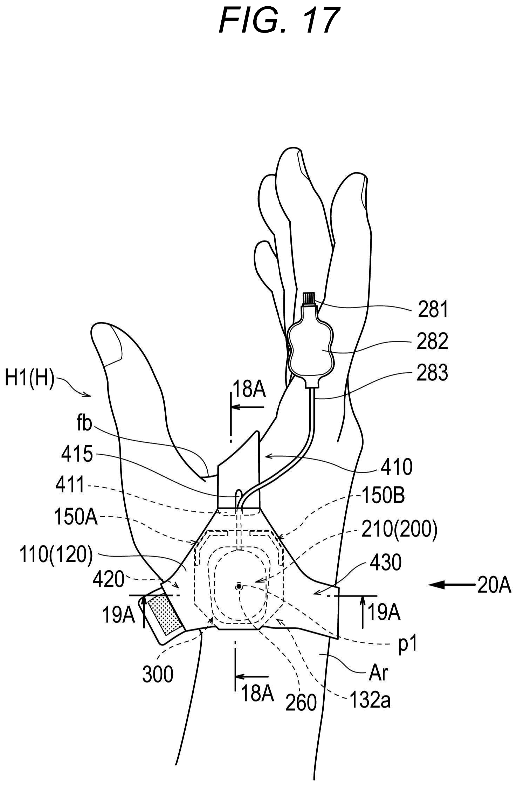

As illustrated in Fig. 17, the first band body 410 can be placed so as to be hooked on an interdigital space fb located between the thumb and the index finger of the right hand H1 of the patient in a state where the inflatable member 210 is placed at the first puncture site p1.

As illustrated in Figs. 1, 2, 3, and 4, the second band body 420 includes a first end 421 connected to the main body 110 of the cover member 100, a second end 422 located opposite to the first end 421, and an intermediate portion 423 extending between the first end 421 and the second end 422.

The second direction in which the second band body 420 extends is not particularly limited as long as it is a direction different from the first direction in which the first band body 410 extends.

As illustrated in Figs. 1, 2, 3, and 4, the third band body 430 includes a first end 431 integrally connected to the main body 110 of the cover member 100, a second end 432 located opposite to the first end 431, and an intermediate portion 433 extending between the first end 431 and the second end 432.

The third direction in which the third band body 430 extends is not particularly limited as long as it is different from the first direction in which the first band body 410 extends and the second direction in which the second band body 420 extends.

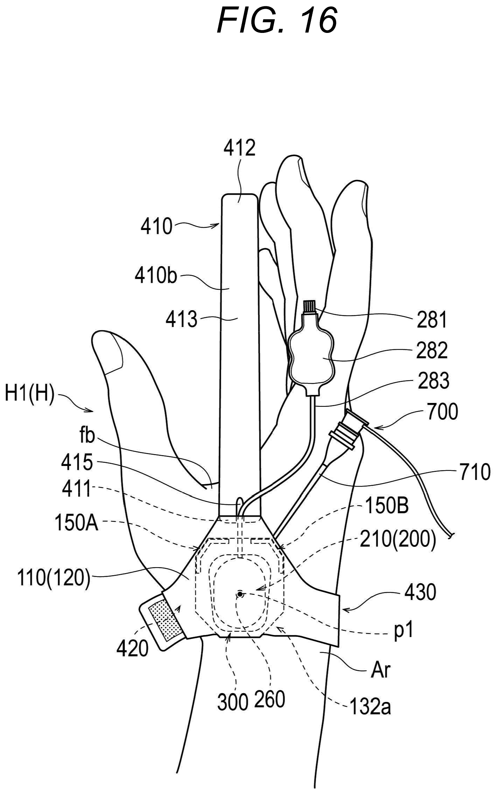

As illustrated in Figs. 16 and 17, the second band body 420 and the third band body 430 can be placed so as to be wrapped along the outer periphery of the right hand H1 when the hemostatic device 10 is worn on the right hand H1 of the patient. As illustrated in Figs. 3 and 4, the second band body 420 and the third band body 430 are located at positions facing each other across the inflatable member 210. Therefore, when the second band body 420 and the third band body 430 are wrapped around the right hand H1, a tensile force in a direction of separating the second band body 420 and the third band body 430 from each other is applied to the both, and a tensile force in the same direction is also applied to the main body 110.

The constituent material of each of the band bodies 410, 420, and 430 is not particularly limited, and each of the band bodies 410, 420, and 430 can be made of, for example, a vinyl chloride resin, a polyurethane resin, a polyester resin, or the like. Furthermore, a shape, a length, a thickness, and the like of each of the band bodies 410, 420, and 430 are not particularly limited.

Four securing parts, that is, a first securing part 510, a second securing part 520, a third securing part 530, and a fourth securing part 540, which enable the cover member 100 to be secured on the right hand H1, are disposed in the band bodies 410, 420, and 430, respectively.

As illustrated in Fig. 1, the first securing part 510 is provided on an outer surface of the intermediate portion 423 of the second band body 420.

As illustrated in Fig. 1, the second securing part 520 is provided on an outer surface of the intermediate portion 433 of the third band body 430.

As illustrated in Fig. 2, the third securing part 530 is provided on an inner surface of the intermediate portion 433 of the third band body 430.

As illustrated in Fig. 2, the fourth securing part 540 is provided on an inner surface of the intermediate portion 413 of the first band body 410.

The first securing part 510 and the second securing part 520 can be constituted by a male side of a hook-and-loop fastener. The third securing part 530 and the fourth securing part 540 can be constituted by a female side of a hook-and-loop fastener. The hook-and-loop fastener in the present specification is a fastener that allows surfaces to be attached and detached, and is, for example, Magic Tape (registered trademark) or Velcro (registered trademark).

The second band body 420 and the third band body 430 are configured to be attachable and detachable by means of the first securing part 510 located on the outer surface of the intermediate portion 423 of the second band body 420 and the third securing part 530 located on the inner surface of the intermediate portion 433 of the third band body 430. The first band body 410 and the third band body 430 are configured to be attachable and detachable via the fourth securing part 540 located on the inner surface of the intermediate portion 413 of the first band body 410 and the second securing part 520 located on the outer surface of the intermediate portion 433 of the third band body 430.

Note that a specific structure of each of the securing parts 510, 520, 530, and 540 is not limited as long as the cover member 100 can be secured on the right hand H1 by connecting the band bodies 410, 420, and 430 to each other in a state where the hemostatic device 10 is placed on the hand H (right hand H1 or left hand). For example, it is possible to freely eliminate some of the securing parts or to freely change the position where the securing part is provided in each of the band bodies 410, 420, and 430. Furthermore, in a case where each of the securing parts 510, 520, 530, and 540 is formed of the hook-and-loop fastener, the male side and the female side of the hook-and-loop fastener may be interchanged. In addition, the securing parts 510, 520, 530, and 540 may be configured using, for example, a coupling mechanism or the like including a frame part provided with a snap, a button, a clip, or a protrusion and an engaged part provided with a hole engageable with the frame part.

### <Support Member 300>

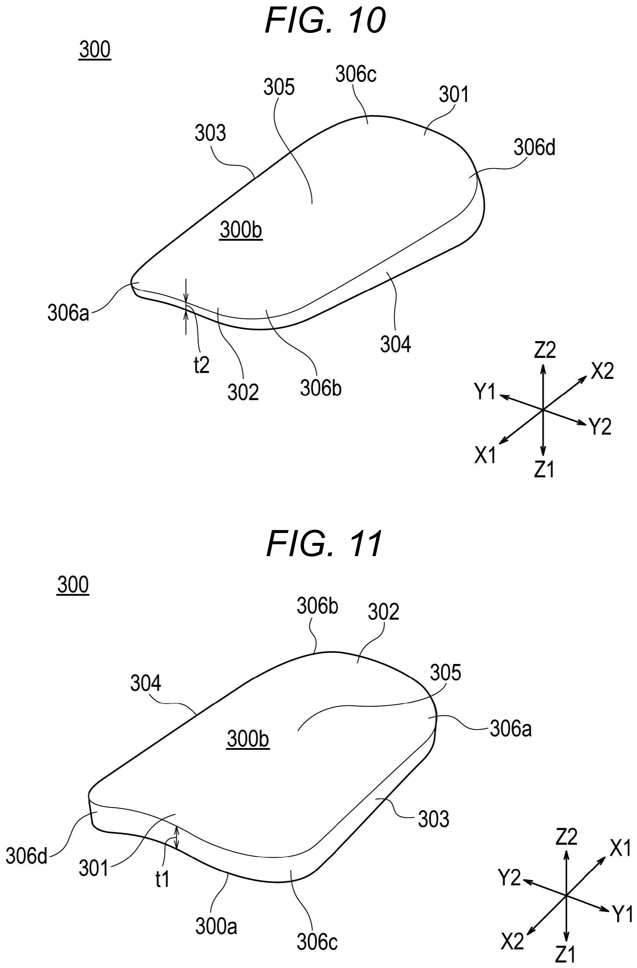

Figs. 10 to 13 illustrate the support member 300 viewed from each direction. In the drawings, arrows X1 and X2 indicate a longitudinal direction of the support member 300 (the same direction as the direction of the longitudinal width wa of the inflatable member 210 illustrated in Fig. 9), arrows Y1 and Y2 indicate a width direction of the support member 300 (the same direction as the direction of the lateral width wb of the inflatable member 210 illustrated in Fig. 9), and arrows Z1 and Z2 indicate a thickness direction of the support member 300.

The support member 300 can be made of a material harder than the inflatable member 210. The support member 300 can be disposed to overlap the inflatable member 210 in the plan view illustrated in Fig. 5. The support member 300 can be inserted into the insertion portion 135 located in the holding portion 130.

As illustrated in Figs. 5, 6, 9, and 10, the support member 300 includes the upper end portion 301 that is located on the first band body 410 side and serves as an end of the support member 300, the lower end portion 302 serving as an end opposite to the upper end portion 301, a pair of side surface portions 303 and 304 connecting the upper end portion 301 and the lower end portion 302, an intermediate portion 305 located between the upper end portion 301 and the lower end portion 302, and a plurality of edge portions 306a, 306b, 306c, and 306d connecting the upper end portion 301 and the intermediate portion 305.

The upper end portion 301 can be placed on the fingertip side (distal side) of the finger in a state where the hemostatic device 10 is worn on the right hand H1. The lower end portion 302 can be placed on the forearm Ar side (proximal side) in the state where the hemostatic device 10 is worn on the right hand H1. Note that the upper end portion 301 defines a distal portion of the support member 300, and the lower end portion 302 defines a proximal portion of the support member 300.

The upper end portion 301 of the support member 300 has a flat portion 301a that is linear in the plan view illustrated in Fig. 9. The flat portion 301a extends substantially linearly between the third edge portion 306c and the fourth edge portion 306d located on the upper end portion 301 side.

The lower end portion 302 of the support member 300 has a flat portion 302a that is linear in the plan view illustrated in Fig. 9. The flat portion 302a extends substantially linearly between the first edge portion 306a and the second edge portion 306b located on the lower end portion 302 side.

As illustrated in Fig. 9, the support member 300 is configured such that a width of the support member 300 decreases from the upper end portion 301 to the lower end portion 302. Therefore, the width ws2 of the lower end portion 302 of the support member 300 is smaller than a width ws1 of the upper end portion 301.

In the present embodiment, the width ws1 of the upper end portion 301 of the support member 300 is defined by a dimension (maximum width) of a section having the largest width in a region located on the upper end portion 301 side with respect to the intermediate portion 305 of the support member 300.

The support member 300 has a substantially trapezoidal shape in which the width ws1 of the upper end portion 301 is larger than the width ws2 of the lower end portion 302. The center of gravity G of the outer shape of the support member 300 is located at a position shifted from the intermediate portion 305 of the support member 300 to the upper end portion 301 (to the lower base of the trapezoidal shape) by a predetermined distance.

As illustrated in Figs. 7, 10, and 11, the upper surface 300b of the support member 300 is curved toward the second band body 420 and the third band body 430. That is, the upper surface 300b of the support member 300 has a vertex protruding most near the center in the width direction of the support member 300. Furthermore, the vertex of the upper surface 300b of the support member 300 is located along the longitudinal direction of the support member 300. That is, the vertex of the upper surface 300b of the support member 300 is located along the longitudinal direction passing through the intermediate portion 305.

The upper surface 300b of the support member 300 can be formed to have a substantially constant radius of curvature at each section in the longitudinal direction of the support member 300, for example.

As illustrated in Figs. 7, 11, and 12, the lower surface 300a of the support member 300 is curved to project in a direction away from the inflatable member 210. Similarly to the upper surface 300b, the lower surface 300a is curved toward the second band body 420 and the third band body 430.

The lower surface 300a of the upper end portion 301 of the support member 300 can be formed to have a radius of curvature smaller than a radius of curvature of the lower surface 300a of the lower end portion 302 of the support member 300, for example.

As illustrated in Figs. 10, 11, and 12, the support member 300 is configured such that a thickness of each of the side surface portions 303 and 304 connecting the upper end portion 301 and the lower end portion 302 is reduced from the upper end portion 301 to the lower end portion 302. That is, the support member 300 is inclined such that a distance between the upper surface 300b and the lower surface 300a decreases from the upper end portion 301 to the lower end portion 302 in the cross-sectional view illustrated in Fig. 6 and the cross-sectional view illustrated in Fig. 18.

The support member 300 has a shape bilaterally symmetric with respect to the center line C of the first band body 410 in the main body 110. In the present embodiment, the support member 300 has a bilaterally symmetrical shape with respect to a line segment that is orthogonal to an imaginary axis along the width ws1 of the upper end portion 301 and passes through a point where distances from both ends of the width ws1 of the upper end portion 301 are equal in the plan view illustrated in Fig. 9. Therefore, the support member 300 can be disposed on the main body 110 so as to be bilaterally symmetrical with respect to the center line C of the first band body 410. Furthermore, as illustrated in the plan view in Fig. 9, the side surface portions 303 and 304 are formed to be bilaterally symmetrical with respect to the center line C of the first band body 410 in the main body 110. The side surface portions 303 and 304 extend to be bilaterally symmetrical such that the width of the support member 300 decreases at a substantially constant rate from the upper end portion 301 side to the lower end portion 302 side.

Each of the edge portions 306a, 306b, 306c, and 306d located at the four corners of the support member 300 is formed in a curved shape that is rounded.

The hemostatic device 10 is preferably formed such that a section of the inflatable member 210 where the marker portion 260 is provided, a section of the cover member 100 (the upper surface region 131 and the lower surface region 132 of the holding portion 130) overlapping the marker portion 260, and a section of the support member 300 overlapping the marker portion 260 are transparent. In a case where each of the members 100, 210, and 300 is configured in this manner, an operator can easily visually confirm the position of the marker portion 260 provided on the inflatable member 210 and/or the first puncture site p1 through the sections formed to be transparent in the members 100, 210, and 300 when the hemostatic device 10 is worn on the right hand H1 of the patient as illustrated in Figs. 15 to 17. Note that the term "transparent" includes being colored transparent, being colorless transparent, and being translucent.

The support member 300 is made of a material harder than the inflatable member 210. Therefore, when the inflatable member 210 applies a compressive force to the first puncture site p1 as illustrated in Figs. 18 and 19, the support member 300 can press the inflatable member 210 against the right hand H1 of the patient. Accordingly, it is possible to prevent the inflatable member 210 from being lifted from the right hand H1 of the patient.

When the inflatable member 210 is made of the above-described material, examples of a constituent material of the support member 300 include acrylic resin, polyvinyl chloride (particularly hard polyvinyl chloride), polyolefin such as polyethylene, polypropylene, or polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, and polyethylene terephthalate (PET).

### <Fixed Region 150A, 150B>

As illustrated in Figs. 4, 5, and 9, the edge 121 of the lower surface region 132 has fixed regions 150A and 150B fixed to the upper surface region 131.

The edge 121 of the lower surface region 132 has a non-fixed region 160 that is adjacent to each of the fixed regions 150A and 150B and that is not fixed to the upper surface region 131.

The fixed region 150A and the fixed region 150B can be arranged to face each other across the non-fixed region 160 in the plan views illustrated in Figs. 4 and 9, for example. In addition, the fixed region 150A and the fixed region 150B can be formed to be bilaterally symmetrical with respect to the center line C of the first band body 410. The fixed regions 150A and 150B can be constituted by, for example, an adhesive portion or a fused portion that fixes the lower surface region 132 to the sheet material 120.

The fixed regions 150A and 150B are located on the distal side with respect to the support member 300 and are located at positions surrounding the edge portions 306c and 306d, respectively, in plan view.

The wording "located on the distal side with respect to the support member 300 and are located at positions surrounding the edge portions 306c and 306d, respectively, in plan view" means that the edge portions 306c and 306d of the support member 300 are located in a region sandwiched between the two fixed regions 150A and 150B that face each other. In addition, the above definition means that the edge portions 306c and 306d are located such that, when each of the edge portions 306c and 306d has a curved shape as in the present embodiment, and imaginary straight lines connecting inflection points of the edge portions 306c and 306d are extended to the fixed regions 150A and 150B, respectively, the imaginary straight lines intersect the fixed regions 150A and 150B, respectively. Note that, in a case where each of the edge portions 306c and 306d is configured to have a vertex having an acute angle or an obtuse angle, the above definition means that the edge portions 306c and 306d are located such that, when the imaginary straight lines connecting the vertices are extended to the fixed regions 150A and 150B, the imaginary straight lines intersect the fixed regions 150A and 150B, respectively.

The fixed region 150A located on the left side in the plan view illustrated in Fig 9 includes a first fixed region 151 located on the distal side with respect to the upper end portion 301 of the support member 300, and a second fixed region 152 provided continuously with the first fixed region 151 and extending from the first fixed region 151 toward the lower end portion 302 of the support member 300.

The first fixed region 151 of the fixed region 150A extends substantially parallel to the flat portion 301a of the support member 300. Note that the first fixed region 151 of the fixed region 150A constitutes a "left fixed region 151a" disposed on the left side with respect to the extension of the center line C of the first band body 410 in the plan view illustrated in Fig. 9.

The second fixed region 152 of the fixed region 150A includes a first part 152a extending obliquely from the first fixed region 151 toward the lower end portion 302 of the support member 300, and a second part 153a extending substantially linearly from the first part 152a toward the lower end portion 302 of the support member 300.

The fixed region 150B located on the right side in the plan view illustrated in Fig 9 includes a first fixed region 151 located on the distal side with respect to the upper end portion 301 of the support member 300, and a second fixed region 152 provided continuously with the first fixed region 151 and extending from the first fixed region 151 toward the lower end portion 302 of the support member 300.

The first fixed region 151 of the fixed region 150B extends substantially parallel to the flat portion 301a of the support member 300. Note that the first fixed region 151 of the fixed region 150B constitutes a "right fixed region 151b" disposed on the right side with respect to the extension of the center line C of the first band body 410 in the plan view illustrated in Fig. 9. As illustrated in Fig. 5, the right fixed region 151b is disposed so as to face the left fixed region 151a across the extension of the slit 415.

The second fixed region 152 of the fixed region 150B includes a first part 152b extending obliquely from the first fixed region 151 toward the lower end portion 302 of the support member 300, and a second part 153b extending substantially linearly from the first part 152b toward the lower end portion 302 of the support member 300.

As illustrated in Fig. 9, the holding portion 130 has the clearance 157 between the holding portion 130 and the support member 300 in plan view.

The width wg of the clearance 157 can be equal to or larger than the thickness t1 (see Fig. 11) of the upper end portion of the support member 300.

The above-mentioned "width wg of the clearance 157" can be defined by a linear distance between the upper end portion 301 (flat portion 301a) of the support member 300 and the first fixed region 151 of each of the fixed regions 150A and 150B.

As illustrated in Fig. 5, the second band body 420 and the third band body 430 extend in the second direction and the third direction, respectively, from the main body 110 of the cover member 100 via the second fixed regions 152 located between the main body 110 and the second and third band bodies 420 and 430.

The wording "the second band body 420 (third band body 430) extends in the second direction (third direction) from the main body 110 of the cover member 100 via the second fixed region 152 located between the main body 110 and the second band body 420 (third band body 430)" means that at least a part of the second fixed region 152 is located between the main body 110 of the cover member 100 where the inflatable member 210 is located and the second band body 420 (third band body 430).

As illustrated in Fig. 5, the second band body 420 has a deflected portion 425 located on the first band body 410 side with respect to an end 154a (lower end of the second part 153a), which is located on the curved region 133 side, of the second fixed region 152 of the fixed region 150A in plan view.

As illustrated in Fig. 5, the third band body 430 has a deflected portion 435 located on the first band body 410 side with respect to an end 154b (lower end portion of the second part 153b), which is located on the curved region 133 side, of the second fixed region 152 of the fixed region 150B in plan view.

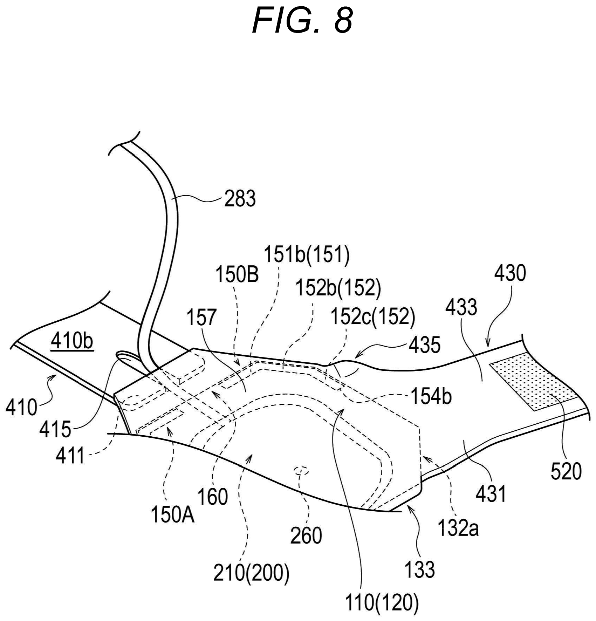

Each of the deflected portions 425 and 435 is defined by a section of a member constituting each of the band bodies 420 and 430 where the member is deflected so as to be rippled in the thickness direction of each of the band bodies 420 and 430 (see Fig. 8). Each of the deflected portions 425 and 435 is configured to easily extend along the direction in which a tensile force, which is externally applied, is applied as compared with the sections of the band bodies 420 and 430 where the deflected portions 425 and 435 are not formed.

The maximum width of the support member 300 is set larger than the maximum width wh (see Fig. 5) of the holding portion 130 between the curved region 133 and the ends (lower ends of the second parts 153a and 153b) 154a and 154b of the second fixed regions 152 on the curved region 133 side.

In the present embodiment, the maximum width of the support member 300 is the width ws1 (see Fig. 9) of the upper end portion 301 of the support member 300.

The "maximum width wh of the holding portion 130" can be defined by the length of the shortest straight line connecting the curved region 133 and the end 154a (or the end 154b) of the second fixed region 152 on the curved region 133 side in the plan view illustrated in Fig. 9.

In the hemostatic device 10, the maximum width of the support member 300 (the width ws1 of the upper end portion 301) is larger than the maximum width wh of the holding portion 130, whereby it is possible to more reliably prevent the support member 300 from slipping out of the clearance between the fixed regions 150A and 150B and the curved region 133.

As illustrated in Fig. 9, the ends (lower ends of the second parts 153a and 153b) 154a and 154b, which are located on the curved region 133 side, of the fixed regions 150A and 150B can be disposed so as to be located on the first band body 410 side with respect to the intermediate portion 305 of the support member 300.

As illustrated in Figs. 5 and 6, the first end 411 of the first band body 410 can be connected to the inner surface 110a side of the main body 110 of the cover member 100 while being spaced apart from the first fixed region 151.

As illustrated in Fig. 9, the width wn of the non-fixed region 160 can be set smaller than the width ws1 of the upper end portion 301 of the support member 300. In the hemostatic device 10, the width wn of the non-fixed region 160 is smaller than the width ws1 of the upper end portion 301 of the support member 300, whereby it is possible to prevent the support member 300 from slipping out of the holding portion 130 through the non-fixed region 160.

### <Injection Member 281>

The injection member 281 includes a connector having an incorporated check valve (not illustrated). A syringe (not illustrated) can be connected to the injection member 281. The injection member 281 is connected to the inflatable member 210 using the tube member 283.

A buffer member 282 having an inflatable space is disposed between the injection member 281 and the inflatable member 210. The buffer member 282 is constituted by a flexible bag-shaped member in which a space is formed. Note that the buffer member 282 may be provided with an arrow-shaped marker indicating an insertion direction of the syringe into the injection member 281.

The injection member 281 is connected to one end of the buffer member 282. A lumen of the injection member 281 communicates with the space of the buffer member 282. However, while the check valve incorporated in the injection member 281 is closed, communication between the lumen of the injection member 281 and the space of the buffer member 282 is blocked.

The tube member 283 having flexibility is connected to the other end of the buffer member 282. A lumen of the tube member 283 communicates with the space of the buffer member 282. Furthermore, the other end of the tube member 283, opposite to the one end connected to the buffer member 282, is connected to the inflatable member 210. The lumen of the tube member 283 communicates with the inner cavity 210a of the inflatable member 210.

To inflate the inflatable member 210, the operator inserts a distal tubular portion of the syringe (not illustrated) into the injection member 281 to open the check valve. The operator injects air in the syringe into the inner cavity 210a of the inflatable member 210 by pushing a plunger of the syringe in a state where the check valve of the injection member 281 is open.

When the air is injected into the inner cavity 210a of the inflatable member 210, the inflatable member 210 is inflated. When the inflatable member 210 is inflated, the buffer member 282 communicating with the inner cavity 210a of the inflatable member 210 by means of the tube member 283 expands. The operator can easily understand that the inflatable member 210 is inflated without leakage of air by visually confirming expansion of the buffer member 282.

To deflate the inflatable member 210, the operator inserts the distal tubular portion of the syringe into the injection member 281 and pulls the plunger of the syringe. By performing the operation described above, the operator can discharge the air in the inner cavity 210a of the inflatable member 210 to the syringe.

Note that the injection member 281, the buffer member 282, and the tube member 283 may be prepared and provided in a state of being coupled to the inflatable member 210, or may be prepared and provided in a state of being separated from the inflatable member 210. In addition, the injection member 281 is not particularly limited as to a specific configuration or the like as long as the supply of the fluid to the inner cavity 210a of the inflatable member 210 and the discharge of the fluid from the inner cavity 210a of the inflatable member 210 can be controlled.

### <Usage Example of Hemostatic Device>

Next, a usage example and operational effects of the hemostatic device 10 will be described with reference to Figs. 14 to 19.

Hereinafter, a procedure of using the hemostatic device 10 to stop bleeding at the first puncture site p1 formed in the right hand H1 of the patient illustrated in Fig. 14 will be described.

Fig. 15 illustrates a state where various procedures performed using the sheath tube 610 of the introducer 600 inserted into the first puncture site p1 are completed.

When placing the hemostatic device 10 on the right hand H1 of the patient, the operator places the inflatable member 210 and the support member 300 so as to overlap the first puncture site p1 as illustrated in Fig. 15. At this time, the operator can appropriately position the inflatable member 210 and the support member 300 at the first puncture site p1 by placing the marker portion 260 at the first puncture site p1 while visually confirming the position of the marker portion 260 provided on the inflatable member 210.

When placing the hemostatic device 10 on the right hand H1 of the patient, the operator can place the support member 300 so as to overlap at least a part of the metacarpal bone B1 of the index finger and at least a part of the metacarpal bone B2 of the thumb. For example, when stopping bleeding at the first puncture site p1, the operator can place the support member 300 at a predetermined position P1 located on the extension of the metacarpal bone B1 and the metacarpal bone B2 as illustrated in Fig. 14. In addition, when stopping bleeding at the second puncture site p2, the operator can place the support member 300 at a predetermined position P2 located on the extension of the metacarpal bone B1 and the metacarpal bone B2 and on the forearm A side with respect to the predetermined position P1 as illustrated in Fig. 14.

An interval between the metacarpal bone B1 of the index finger and the metacarpal bone B2 of the thumb gradually decreases from the fingertip side to the forearm Ar side (see Fig. 14). The width of the support member 300 decreases from the upper end portion 301 positioned on the fingertip side to the lower end portion 302 positioned on the forearm Ar side (see Fig. 9). Therefore, the operator can position the support member 300 along at least a part of the metacarpal bone B1 of the index finger and at least a part of the metacarpal bone B2 of the thumb by placing the support member 300 so as to overlap the metacarpal bone B1 of the index finger and the metacarpal bone B2 of the thumb from the dorsal side of the right hand H1 of the patient. The operator can prevent the formation of a gap between the inflatable member 210 located on the lower surface 300a side of the support member 300 and the right hand H1 by placing the support member 300 along at least a part of the metacarpal bone B1 of the index finger and at least a part of the metacarpal bone B2 of the thumb.

As illustrated in Fig. 16, the operator wraps the second band body 420 and the third band body 430 along the outer periphery of the right hand H1 of the patient.

In the hemostatic device 10, the second band body 420 and the third band body 430 have deflected portions 435 located on the first band body 410 side with respect to the ends 154a and 154b, which are located on the curved region 133 side, of the second fixed regions 152 of the fixed regions 150A and 150B in plan view (see Figs. 5 and 8). The hemostatic device 10 has the deflected portions 425 and 435, whereby the vicinity of the first ends 421 and 431 of the band bodies 420 and 430 is easily stretched. Therefore, in the hemostatic device 10, the range of movement of the band bodies 420 and 430 in the vicinity of the first ends 421 and 431 is increased. The operator can easily wrap the band bodies 420 and 430 around the right hand H1 by deforming the deflected portions 425 and 435.

In addition, when the patient moves the right hand H1 while the operator wraps the band bodies 420 and 430 around the right hand H1, a force for pressing the band bodies 420 and 430 against the right hand H1 may be applied to the right hand H1. When such a force is applied to the right hand H1 of the patient, there is a possibility that each of the band bodies 420 and 430 digs into the right hand H1 to give the patient a feeling of pain. In the hemostatic device 10, the deflected portions 425 and 435 are provided in the band bodies 420 and 430 as described above. Therefore, when the band bodies 420 and 430 are wrapped around the right hand H1, the band bodies 420 and 430 are deformed so as to follow the surface of the right hand H1 with the deflected portions 425 and 435 as base points, so that the force applied to the right hand H1 of the patient can be released. Therefore, the hemostatic device 10 can prevent the patient from feeling pain or the like when the band bodies 420 and 430 are wrapped around the right hand H1.

In the hemostatic device 10, the ends 154a and 154b, which are located on the curved region 133 side, of the fixed regions 150A and 150B are positioned on the first band body 410 side with respect to the intermediate portion 305 of the support member 300. Therefore, the hemostatic device 10 can ensure the mobility in the vicinity of the first ends 421 and 431 of the band bodies 420 and 430 when the band bodies 420 and 430 are wrapped around the right hand H1. Accordingly, it is possible to wrap the band bodies 420 and 430 of the hemostatic device 10 around the right hand H1 more easily and smoothly.

The operator can connect the second band body 420 and the third band body 430 by means of the securing parts 510 and 530 by bringing the third securing part 530 (see Fig. 2) provided on the inner surface of the third band body 430 into contact with the first securing part 510 (see Fig. 1) provided on the outer surface of the second band body 420. The operator can firmly wrap the band bodies 420 and 430 along the outer periphery of the right hand H1 by connecting the band bodies 420 and 430 to each other in a state where the support member 300 overlaps at least a part of the metacarpal bone B1 of the index finger and at least a part of the metacarpal bone B2 of the thumb.

The upper surface 300b of the support member 300 is curved toward the second band body 420 (toward the side surface portion 303) and the third band body 430 (the side surface portion 304) (see Figs. 7 and 10). Therefore, the operator can press the support member 300 against the right hand H1 of the patient from the upper surface 300b side along the outer periphery of the right hand H1 as illustrated in Fig. 19 by wrapping the second band body 420 and the third band body 430 around the right hand H1 as illustrated in Fig. 16. Thus, the hemostatic device 10 can increase the securing force for securing the support member 300 and the inflatable member 210 to the right hand H1 of the patient.

As illustrated in Fig. 17, the operator places a part of the first band body 410 on a palm side of the right hand H1 of the patient while passing the first band body 410 through the interdigital space fb located between the thumb and the index finger of the right hand H1 of the patient. When doing so, the operator brings the fourth securing part 540 (see Fig. 2) provided on the inner surface of the first band body 410 into contact with the second securing part 520 (see Fig. 1) provided on the outer surface of the third band body 430, thereby being capable of connecting the first band body 410 and the third band body 430 by means of the securing parts 520 and 540.

In the hemostatic device 10, the first end 411 of the first band body 410 is connected to the inner surface 110a side of the main body 110 of the cover member 100 while being spaced apart from the first fixed region 151 (see Figs. 5 and 6). As described above, the first band body 410 is spaced apart from the first fixed region 151. Therefore, when the first band body 410 of the cover member 100 is placed in the interdigital space fb, the first fixed region 151 and the first end 411 function as two-step joints. Therefore, the hemostatic device 10 can increase the mobility of the first band body 410 when the first band body 410 is placed in the interdigital space fb. In addition, since the first band body 410 is spaced apart from the first fixed region 151, the mobility in the lateral direction in the vicinity of the first end 411 can also be ensured. As a result, the first band body 410 of the hemostatic device 10 can be more easily wrapped around the interdigital space fb. The first band body 410 of the hemostatic device 10 can be deflected so as to follow the surface of the right hand H1, whereby it is possible to prevent the hemostatic device 10 from being bulky in a state of being worn on the right hand H1 of the patient. Therefore, it is possible to suitably prevent the hemostatic device 10 from coming into contact with another article or the like in a state of being worn on the right hand H1 of the patient.

Note that, if no space is provided between the first band body 410 and the first fixed region 151, the section (for example, a fused portion) to which the first end 411 is fixed and the first fixed region 151 are arranged at the same position or close to each other, so that the vicinity of the first end 411 becomes rigid to impair the mobility in the vicinity of the first end 411.

In the hemostatic device 10, the tube member 283 passes through the non-fixed region 160 between the left fixed region 151a and the right fixed region 151b, and is inserted from the inner surface 410a side of the first band body 410 toward the outer surface 410b side of the first band body 410 through the slit 415 (see Fig. 5). Therefore, the operator can prevent the tube member 283 from unnecessarily moving by locating a part of the tube member 283 between the left fixed region 151a and the right fixed region 151b and in the slit 415 when placing the hemostatic device 10 on the right hand H1 of the patient. This improves operability when the operator handles the hemostatic device 10. In addition, in a state where the hemostatic device 10 is placed on the right hand H1 of the patient, the tube member 283 is inserted between the left fixed region 151a and the right fixed region 151b and the slit 415, and a part of the tube member 283 is drawn out and placed on the outer surface 410b side of the first band body 410, whereby the tube member 283 can be prevented from being pressed against the right hand H1 of the patient by the first band body 410 placed in the interdigital space fb.

The support member 300 includes the upper end portion 301 and the side surface portions 303 and 304. The first band body 410 extends from the upper end portion 301 side, the second band body 420 extends from the side surface portion 303 side, and the third band body 430 extends from the side surface portion 304 side (see Fig. 5). Therefore, the support member 300 of the hemostatic device 10 can be secured to the right hand H1 of the patient in a state in which a uniform force is applied to the positions corresponding to the portions 301, 303, and 304 of the support member 300 by connecting the band bodies 410, 420, and 430 to each other.

The support member 300 is bilaterally symmetrical with respect to the center line C of the first band body 410 in the main body 110 (see Fig. 9). Therefore, the hemostatic device 10 can uniformly apply a force to the support member 300 along a bilaterally symmetrical direction by wrapping the band bodies 420 and 430 bilaterally symmetrically extending based on the first band body 410 around the right hand H1 of the patient. Accordingly, the hemostatic device 10 can prevent displacement of the support member 300 in a state where the support member 300 is placed on the right hand H1 of the patient.

The right hand H1 of the patient has an inclined part in each section (for example, the vicinity of the snuff box Sb). Furthermore, a shape of the inclined part of the right hand H1 depends on individual differences of patients. For example, in a case where the lower surface 300a of the support member 300 is formed in a flat shape, it is difficult to place the support member 300 along the inclined part of the right hand H1. In the hemostatic device 10 according to the present embodiment, the lower surface 300a of the support member 300 is curved to project in a direction away from the inflatable member 210 (see Figs. 7 and 19). Therefore, when the lower surface 300a of the support member 300 is placed so as to overlap the inclined part of the right hand H1 of the patient, the lower surface 300a of the support member 300 can be placed along the right hand H1 of the patient. As a result, the support member 300 of the hemostatic device 10 can be placed to overlap the first puncture site p1 without depending on individual difference of the right hand H1 and the left hand of the patient or the way of placement by the operator. Thus, the support member 300 of the hemostatic device 10 can be appropriately placed at a desired position near the first puncture site p1.

Further, the lower surface 300a of the support member 300 of the hemostatic device 10 is placed along the body surface of the right hand H1 when the operator wraps the second band body 420 and the third band body 430 around the right hand H1 of the patient, whereby the band bodies 420 and 430 extending from the side surface portions 303 and 304 of the support member 300 can be placed close to or in close contact with the body surface of the right hand H1 of the patient (see Fig. 19). Thus, the hemostatic device 10 can increase the securing force for securing the support member 300 and the inflatable member 210 to the right hand H1 of the patient. As a result, even when an impact or the like is applied from the outside in a state where the hemostatic device 10 is worn on the right hand H1 of the patient, the support member 300 and the inflatable member 210 are less likely to be displaced.

The operator inflates the inflatable member 210 by injecting air into the inflatable member 210 in a state where the syringe is connected to the injection member 281. As illustrated in Figs. 18 and 19, when the inflatable member 210 is inflated in the hemostatic device 10, the inflatable member 210 applies a compressive force to the first puncture site p1.

When the inflatable member 210 is inflated in a state where the hemostatic device 10 is worn on the right hand H1 of the patient, the support member 300 presses the inflatable member 210 against the right hand H1 of the patient. As a result, the hemostatic device 10 can prevent the inflatable member 210 from being lifted from the right hand H1 of the patient.

When stopping bleeding using the hemostatic device, the operator can more strongly tighten, for example, the first band body placed in the interdigital space fb so as to increase a securing force to secure the support member to the right hand H1 of the patient. By strongly tightening the first band body, the operator can firmly secure the support member to the right hand H1 of the patient even when the support member is placed in an unstable state on the right hand H1 of the patient. However, when the first band body is strongly tightened, the patient may feel pain. Therefore, in a case where the first band body cannot be strongly tightened due to pain or the like of the patient, the hemostatic device may be placed such that the lower surface of the support member is inclined with respect to the body surface of the right hand H1 of the patient. Specifically, the upper end portion of the support member on which the first band body is disposed is lifted to a position excessively away from the body surface of the right hand H1 of the patient as compared with the lower end portion located opposite to the upper end portion. As a result, in the hemostatic device, the support member is placed to be inclined such that a distance between the lower surface of the support member and the body surface of the right hand H1 of the patient gradually decreases from the upper end portion to the lower end portion of the support member.

The hemostatic device 10 is configured such that the thickness of each of the side surface portions 303 and 304 of the support member 300 is decreased from the upper end portion 301 to the lower end portion 302. In addition, the first band body 410 configured to be placed at the interdigital space fb located between the fingers of the patient extends from the upper end portion 301 side of the support member 300, and is configured to press the upper end portion 301 side of the support member 300 against the right hand H1 of the patient. In the hemostatic device 10 configured as described above, when the inflatable member 210 starts to be inflated in a state where the support member 300 and the inflatable member 210 are secured to the right hand H1 of the patient, the inflatable member 210 applies a force to the vicinity of the upper end portion 301 of the support member 300. The lower end portion 302 side of the support member 300 is lifted with the upper end portion 301 side being pressed against the right hand H1 by the force applied by the inflatable member 210. As a result, the support member 300 is placed such that the lower surface 300a of the support member 300 is substantially parallel with the body surface of the right hand H1 of the patient in a state where the inflatable member 210 is inflated as illustrated in Fig. 18. Therefore, the hemostatic device 10 can apply a compressive force in a direction perpendicular to the first puncture site p1 when the inflatable member 210 is inflated.

The width ws2 of the lower end portion 302 of the support member 300 is smaller than the width ws1 of the upper end portion 301 of the support member 300 (see Fig. 9). Therefore, when the support member 300 is placed on the right hand H1, the support member 300 can be placed along at least a part of the metacarpal bone B1 of the index finger and at least a part of the metacarpal bone B2 of the thumb. Accordingly, the second band body 420 and the third band body 430 of the hemostatic device 10 can be firmly wrapped along the outer periphery of the right hand H1. Further, in the hemostatic device 10, the lower surface 300a of the support member 300 is curved to project in a direction away from the inflatable member 210. Therefore, the inflatable member 210 is curved so as to fit along the lower surface 300a of the support member 300 in a state where the hemostatic device 10 is worn on the right hand H1 of the patient. Thus, the hemostatic device 10 can effectively increase the compressive force applied to the first puncture site p1 by the inflatable member 210.

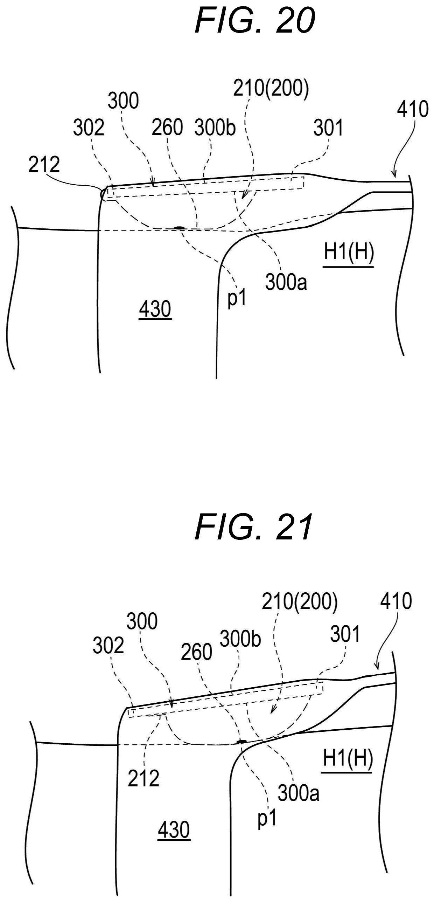

In the hemostatic device 10, the edge 212 located at the lower end portion 302 of the support member 300 in the sheet material constituting the inflatable member 210 is connected to the curved region 133 formed by the cover member 100 (see Figs. 6 and 18). Therefore, the inflatable member 210 is placed such that the center c1 of the inflatable member 210 is located at a position shifted to the proximal portion 122 of the main body 110 in the longitudinal width direction (length in the vertical direction in Fig. 9) of the main body 110. In the hemostatic device 10, the second band body 420 and the third band body 430 having a function of mainly applying a securing force for securing the inflatable member 210 to the right hand H1 are positioned on the proximal portion side (curved region 133 side) of the main body 110 of the cover member 100. Therefore, when the center c1 of the inflatable member 210 is displaced to the position on the proximal portion side of the main body 110 of the cover member 100, a securing force is easily applied from each of the band bodies 410 and 420 to the vicinity of the center c1 of the inflatable member 210 when the band bodies 410 and 420 are wrapped around the right hand H1. As a result, as illustrated in Fig. 20, the distal portion side (first band body 410 side) of the inflatable member 210 is less likely to be lifted, and thus, the compressive force applied to the first puncture site p1 by the inflatable member 210 can be increased.

If the center c1 of the inflatable member 210 is located at a position on the distal portion side of the main body 110 of the cover member 100, a strong tightening force is applied to a section on the proximal portion side of the inflatable member 210 when the band bodies 410 and 420 are wrapped around the right hand H1. As a result, as illustrated in Fig. 21, the section on the distal side of the inflatable member 210 is placed in a state of being lifted from the right hand H1, so that the compressive force to be applied to the first puncture site p1 by the inflatable member 210 may decrease. In addition, since the hemostatic device 10 that is worn on the right hand H1 is placed in a state of being lifted, the possibility that the hemostatic device 10 comes into contact with another article or the like increases.

The lower surface 300a of the support member 300 is curved to project in a direction away from the inflatable member 210 (see Fig. 7). As illustrated in Fig. 19, the hemostatic device 10 is placed such that the support member 300 is along the body surface of the right hand H1 of the patient in a state where the inflatable member 210 is inflated. When the inflatable member 210 is inflated, the hemostatic device 10 presses the inflatable member 210 along the body surface of the right hand H1 of the patient by the lower surface 300a of the support member 300. As a result, in the hemostatic device 10, the inflatable member 210 applies a compressive force in a direction toward the first puncture site p1 from a center side in the width direction of the lower surface 300a of the support member 300 and the side surface portions 303 and 304 located at both ends in the width direction of the lower surface 300a of the support member 300. Therefore, the hemostatic device 10 can effectively increase the compressive force applied to the first puncture site p1 by the inflatable member 210 as compared with a case where the lower surface 300a of the support member 300 is formed in a flat surface shape.

The hemostatic device 10 includes the constricted portion 132a (see Figs. 5 and 9) configured such that the width of the lower surface region 132 decreases from the first band body 410 side toward the curved region 133 side. The constricted portion 132a is not fixed to the upper surface region 131.

In a state where the hemostatic device 10 is worn on the right hand H1, the deformation of the upper surface region 131 is not inhibited by the lower surface region 132 due to the constricted portion 132a located in the lower surface region 132, and the upper surface region 131 can be deformed along the support member 300. Therefore, in a state where the hemostatic device 10 is worn on the right hand H1, the proximal portion 122 of the cover member 100 of the hemostatic device 10 is deformed along both ends of the support member 300 in the width direction, so that a width of the proximal portion 122 of the cover member 100 can be reduced. Since the width of the proximal portion 122 of the cover member 100 decreases, the hemostatic device 10 can prevent an article or the like from being easily caught on the proximal portion 122 of the cover member 100. In particular, in the hemostatic device 10, the constricted portion 132a is not fixed to the upper surface region 131, whereby it is possible to prevent the cover member 100 from being hardly deformed in the vicinity of the constricted portion 132a. Accordingly, it is possible to effectively prevent the hemostatic device 10 from being bulky in a state where the hemostatic device 10 is worn on the right hand H1.

In the hemostatic device 10, the thickness of the support member 300 on the lower end portion 302 side is thinner than the thickness on the upper end portion 301 side (see Fig. 18). Therefore, when the hemostatic device 10 comes into contact with an article such as a table from the lower end portion 302 side of the support member 300 in a state where the hemostatic device 10 is worn on the right hand H1 of the patient, an impact at the time of the contact can be released from the lower end portion 302 side to the upper end portion 301 side. As a result, it is possible to effectively prevent the hemostatic device 10 from being displaced as the article or the like is caught on the lower end portion 302 of the hemostatic device 10.

After the inflatable member 210 is inflated, the operator removes the sheath tube 610 of the introducer 600 from the first puncture site p1 as illustrated in Fig. 17. The operator confirms that there is no bleeding from the first puncture site p1 while stopping bleeding using the hemostatic device 10. In a case where there is bleeding from the first puncture site p1, the operator can adjust an injection amount of air into the inflatable member 210.

In a state where the hemostatic device 10 is worn on the right hand H1 of the patient, the curved region 133 is placed on the forearm Ar side as illustrated in Fig. 17. When the patient moves to twist the wrist to the dorsal side of the right hand H1, the curved region 133 formed by folding back the main body 110 from the proximal side to the distal side comes into contact with the body surface. When the patient moves to twist the wrist or the like, the curved region 133 of the hemostatic device 10 formed in a curved shape comes into contact with the body surface of the wrist, the forearm Ar, or the like of the patient, and thus, it is possible to prevent the proximal portion 122 of the cover member 100 from digging into the body surface of the wrist, the forearm Ar, or the like of the patient.

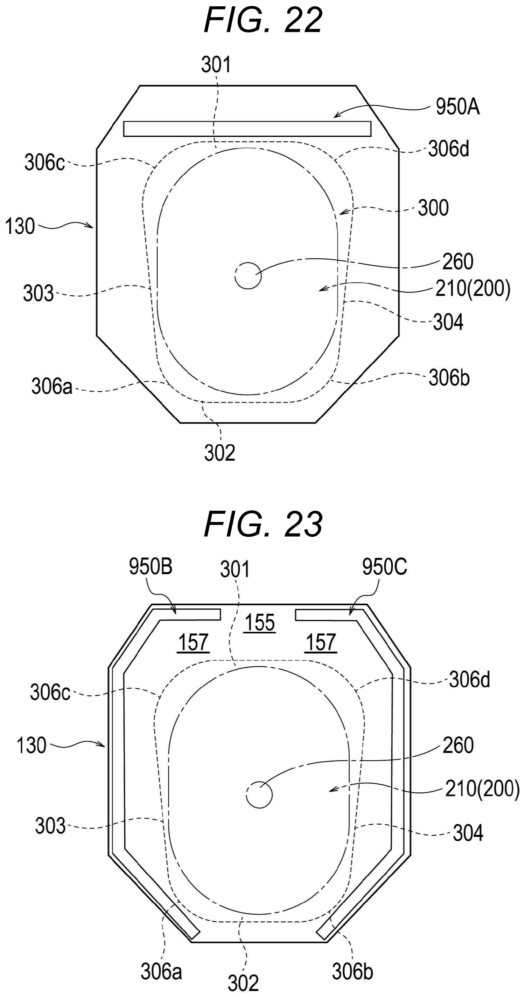

Here, the operational effect of the hemostatic device 10 according to the present embodiment will be described with reference to a comparative example illustrated in Figs. 22 and 23.

In the hemostatic device 10 according to the present embodiment, the edge 121 of the lower surface region 132 has the fixed regions 150A and 150B fixed to the upper surface region 131. In addition, the fixed regions 150A and 150B are located on the distal side with respect to the support member 300 and are located at positions surrounding the edge portions 306c and 306d of the support member 300 in plan view. As described above, the fixed regions 150A and 150B are located at positions surrounding the edge portions 306c and 306d of the support member 300, whereby it is possible to suitably prevent the support member 300 held in the holding portion 130 from slipping out of the holding portion 130 from the first band body 410 side (distal side). In addition, the fixed regions 150A and 150B are located at positions surrounding the edge portions 306c and 306d of the support member 300 as described above, whereby it is possible to suitably prevent the edge portions 306c and 306d located on the distal side of the support member 300 from being lifted via the upper surface region 131.

As an example in which the fixed regions 150A and 150B are not disposed at positions surrounding the edge portions 306c and 306d of the support member 300, it is conceivable that a fixed region 950A is disposed substantially linearly along the upper end portion 301 of the support member 300 as in the comparative example illustrated in Fig. 22. In a case where the fixed region 950A is disposed as described above, the fixed region 950A needs to be disposed at a position as close as possible to the upper end portion 301 of the support member 300 in order to prevent the support member 300 from slipping out of the holding portion 130. However, when the fixed region 950A is fused to a position close to the upper end portion 301 of the support member 300, a corner rising substantially perpendicularly to the thickness direction of the support member 300 is formed in the fixed region 950A due to the thickness of the upper end portion 301 of the support member 300. Therefore, in a state where the hemostatic device 10 is worn on the right hand H1, the section where the corner portion is formed is likely to be caught on another article or the like. In addition, when a sufficient distance for forming the clearance 157 is not provided between the upper end portion 301 of the support member 300 and the fixed region 950A (fused region), and the fixed region 950A extends substantially linearly along the upper end portion 301 of the support member 300 to the vicinity of the outer edge of the holding portion 130 as in the comparative example illustrated in Fig. 22, both end portions (portions hardened by fusion) of the fixed region 950A in the extending direction dig into the body surface of the right hand H1 when the first band body 410 is tightened to the interdigital space fb, and the patient may feel pain.

In the hemostatic device 10 according to the present embodiment, each of the fixed regions 150A and 150B has the first fixed region 151 located on the distal side with respect to the upper end portion 301 of the support member 300, and the second fixed region 152 provided continuously with the first fixed region 151 and extending from the first fixed region 151 toward the lower end portion 302 of the support member 300. The holding portion 130 has the clearance 157 between the support member 300 and the first fixed region 151 in plan view. Further, the width wg of the clearance 157 is equal to or larger than the thickness t1 of the upper end portion 301 of the support member 300.

Since the hemostatic device 10 has the clearance 157, the fixed regions 150A and 150B are not disposed at positions close to the upper end portion 301 of the support member 300. Therefore, it is possible to prevent the formation of a corner due to the arrangement of the fixed regions 150A and 150B at positions close to the upper end portion 301 of the support member 300. In particular, in the hemostatic device 10, the width wg of the clearance 157 is equal to or larger than the thickness t1 of the upper end portion 301 of the support member 300, whereby a sufficient space can be provided between the upper end portion 301 of the support member 300 and the first fixed regions 151. Accordingly, it is possible to prevent the formation of a corner rising substantially perpendicularly along the thickness direction of the upper end portion 301 of the support member 300. Thus, it is possible to prevent the cover member 100 from being likely to be caught on another article or the like in a state where the hemostatic device 10 is worn on the right hand H1. In addition, when the hemostatic device 10 is worn on the right hand H1, it is possible to prevent the fixed regions 150A and 150B (portions hardened by fusion) from digging into the body surface of the right hand H1 upon tightening the first band body 410 to the interdigital space fb. In addition, the hemostatic device 10 has the second fixed region 152 extending from the first fixed region 151 toward the lower end portion 302 of the support member 300 as described above, whereby it is possible to prevent the formation of an unnecessary gap between the inner surface 110a and the outer surface 110b of the main body 110 on the first band body 410 side of the side surface portions 303 and 304 of the support member 300. Thus, it is possible to prevent the hemostatic device 10 from being caught on another article or the like in a state where the hemostatic device 10 is worn on the right hand H1. In addition, in the hemostatic device 10, the support member 300 can be held in a state of being loosely surrounded due to the clearance 157 formed between the first and second fixed regions 151 and 152 and the support member 300, whereby the periphery of the support member 300 can be deformed so as to follow the right hand H1 when the hemostatic device 10 is worn on the right hand H1. As a result, it is possible to effectively prevent the hemostatic device 10 from being caught on another article or the like.

Note that, in a case where, for example, fixed regions 950B and 950C are extended to the vicinity of the lower end portion 302 of the support member 300 as illustrated in Fig. 23, the main body 110 of the cover member 100 is hardly bent and becomes bulky. Therefore, when the hemostatic device 10 is worn on the right hand H1, there is a high risk that the hemostatic device 10 hits or is caught on another article.

As described above, the hemostatic device 10 according to the present embodiment includes a cover member 100 configured to cover a first puncture site p1, a pressing member 200 disposed on the cover member 100 and configured to compress the first puncture site p1, and a support member 300 disposed on the cover member 100 so as to cover the pressing member 200, wherein the cover member 100 includes a main body 110 where the pressing member 200 is located, a first band body 410 configured to be placed between fingers of a patient and extending in a first direction from the main body 110, a second band body 420 extending in a second direction different from the first direction from the main body 110, and a third band body 430 facing the second band body 420 across the pressing member 200 and extending from the main body 110 in a third direction different from the first direction and the second direction, the main body 110 has a two-layer structure including a holding portion 130 that holds the support member 300, the holding portion 130 has an upper surface region 131 located on the upper surface 300b side of the support member 300, a lower surface region 132 located on the lower surface 300a side of the support member 300 and facing the upper surface region 131 across the support member 300, and a curved region 133 connecting the upper surface region 131 and the lower surface region 132 on the proximal side of the support member 300, the curved region 133 is formed by folding back a part of the main body 110 toward the first band body 410, the support member 300 includes an upper end portion 301 located on the first band body 410 side and serves as an end of the support member 300, a lower end portion 302 serving as an end opposite to the upper end portion 301, an intermediate portion 305 positioned between the upper end portion 301 and the lower end portion 302, and an edge portion 306c, 306d connecting the upper end portion 301 and the intermediate portion 305, an edge 121 of the lower surface region 132 has a fixed region 150A, 150B fixed to the upper surface region 131, and the fixed region 150A, 150B is positioned on a distal side with respect to the support member 300 and located at a position surrounding the edge portion 306c, 306d in plan view.

In the hemostatic device 10 according to the present embodiment, the main body 110 included in the cover member 100 includes the holding portion 130 having a two-layer structure to hold the support member 300. The holding portion 130 has the curved region 133 formed by folding back a part of the main body 110 toward the first band body 410. In the hemostatic device 10, the curved region 133 of the holding portion 130 comes into contact with a body surface when the patient moves his/her limb in a state where the hemostatic device 10 is worn on the limb of the patient. Since the curved region 133 is formed in a curved shape, the hemostatic device 10 can prevent a proximal portion of the cover member 100 from digging into the body surface of the patient. In the hemostatic device 10, the edge 121 of the lower surface region 132 of the holding portion 130 that holds the support member 300 has the fixed regions 150A and 150B fixed to the upper surface region 131 of the holding portion 130. The fixed regions 150A and 150B are located on the distal side with respect to the support member 300 and are located at positions surrounding the edge portions 306c and 306d that connect the upper end portion 301 and the intermediate portion 305 of the support member 300 in plan view. Therefore, in the hemostatic device 10, the support member 300 can be prevented from slipping out from the lower end portion 302 side to the outside of the holding portion 130 by the curved region 133 formed by folding back a part of the main body 110, and the support member 300 can be prevented from slipping out from the upper end portion 301 side to the outside of the holding portion 130 by the fixed regions 150A and 150B located at the upper end portion 301 of the support member 300. In addition, the fixed regions 150A and 150B are located on the distal side with respect to the support member 300 and are located at positions surrounding the edge portions 306c and 306d that connect the upper end portion 301 and the intermediate portion 305 of the support member 300 in plan view. Therefore, in a state where the hemostatic device 10 is worn on the limb (right hand H1) of the patient, the cover member 100 can suitably prevent the edge portions 306c and 306d of the support member 300 from being lifted via the upper surface region 131 by the fixed regions 150A and 150B located at positions surrounding the edge portions 306c and 306d of the support member 300. Therefore, by preventing the edge portions 306c and 306d located on the distal side of the support member 300 from being lifted, the hemostatic device 10 can prevent a situation in which an article or the like is likely to be caught on the edge portions 306c and 306d (cover member 100 that covers the edge portions 306c and 306d located on the distal side of the support member 300) located on the distal side of the support member 300. Therefore, according to the hemostatic device 10, it is possible to prevent the proximal portion of the cover member 100 from digging into the body surface of the patient wearing the hemostatic device 10, and it is possible to prevent an article or the like from being caught on the cover member 100 in a state where the hemostatic device 10 is worn on the patient while preventing the support member 300 from falling off from the cover member 100.

In addition, in the hemostatic device 10, each of the fixed regions 150A and 150B includes a first fixed region 151 located on the distal side with respect to the upper end portion 301 of the support member 300 and a second fixed region 152 provided continuously with the first fixed region 151 and extending from the first fixed region 151 toward the lower end portion 302 of the support member 300, the holding portion 130 has a clearance 157 between the support member 300 and the first fixed region 151 in plan view, and the width wg of the clearance 157 is equal to or larger than the thickness t1 of the upper end portion 301 of the support member 300.

The hemostatic device 10 configured as described above has the clearance 157, whereby the fixed regions 150A and 150B are not disposed at positions close to the upper end portion 301 of the support member 300. Therefore, it is possible to prevent the formation of a corner due to the arrangement of the fixed regions 150A and 150B at positions close to the upper end portion 301 of the support member 300. In addition, in the hemostatic device 10, the width wg of the clearance 157 is equal to or larger than the thickness t1 of the upper end portion 301 of the support member 300, whereby a sufficient space can be provided between the upper end portion 301 of the support member 300 and the first fixed region 151. Accordingly, the hemostatic device 10 can suitably prevent formation of a corner rising substantially perpendicularly along the thickness direction of the upper end portion 301 of the support member 300. Thus, it is possible to prevent the cover member 100 from being likely to be caught on another article or the like in a state where the hemostatic device 10 is worn on the right hand H1. In addition, when the hemostatic device 10 is worn on the right hand H1, it is possible to prevent the fixed regions 150A and 150B (portions hardened by fusion) from digging into the body surface of the right hand H1 upon tightening the first band body 410 to the interdigital space fb. In addition, the hemostatic device 10 has the second fixed region 152 extending from the first fixed region 151 toward the lower end portion 302 of the support member 300 as described above, whereby it is possible to prevent the formation of an unnecessary gap between the inner surface 110a and the outer surface 110b of the main body 110 on the first band body 410 side of the side surface portions 303 and 304 of the support member 300. Thus, it is possible to prevent the hemostatic device 10 from being caught on another article or the like in a state where the hemostatic device 10 is worn on the right hand H1. In addition, in the hemostatic device 10, the support member 300 can be held in a state of being loosely surrounded due to the clearance 157 formed between the first and second fixed regions 151 and 152 and the support member 300, whereby the periphery of the support member 300 can be deformed so as to follow the right hand H1 when the hemostatic device 10 is worn on the right hand H1. As a result, it is possible to effectively prevent an article or the like from being caught on the hemostatic device 10.

In addition, in the hemostatic device 10, the second band body 420 and the third band body 430 extend from the main body 110 in the second direction and the third direction via the second fixed region 152 located between the second and third band bodies 420 and 430 and the main body 110, and the second band body 420 and the third band body 430 have deflected portions 425 and 435 located on the first band body 410 side with respect to the ends 154a and 154b of the second fixed regions 152 located on the curved region 133 side in plan view.

The hemostatic device 10 has the deflected portions 425 and 435, whereby the vicinity of the first ends 421 and 431 of the band bodies 420 and 430 is easily stretched. Therefore, in the hemostatic device 10, the range of movement of the band bodies 420 and 430 in the vicinity of the first ends 421 and 431 is increased. The operator can easily wrap the band bodies 420 and 430 around the right hand H1 by deforming the deflected portions 425 and 435.

Further, in the hemostatic device 10, the pressing member 200 is an inflatable member 210 inflatable by the injection of a fluid, the inflatable member 210 is connected to an injection member 281 for injecting the fluid into the inflatable member 210 by means of a tube member 283, the first band body 410 has a slit 415 extending in the longitudinal direction of the first band body 410, the first fixed region 151 includes a left fixed region 151a and a right fixed region 151b facing the left fixed region 151a across the extension of the slit 415, and the tube member 283 is located between the left fixed region 151a and the right fixed region 151b in plan view, passes through a non-fixed region 160 in which the edge of the lower surface region is not fixed to the upper surface region, and is inserted from the inner surface 410a side of the first band body 410 toward the outer surface 410b side of the first band body 410 through the slit 415.

In the hemostatic device 10, the tube member 283 passes through the non-fixed region 160 between the left fixed region 151a and the right fixed region 151b, and is inserted from the inner surface 410a side of the first band body 410 toward the outer surface 410b side of the first band body 410 through the slit 415. Therefore, the operator can prevent the tube member 283 from unnecessarily moving by locating a part of the tube member 283 between the left fixed region 151a and the right fixed region 151b and in the slit 415 when placing the hemostatic device 10 on the right hand H1 of the patient. This improves operability when the operator handles the hemostatic device 10. In addition, in a state where the hemostatic device 10 is placed on the right hand H1 of the patient, the tube member 283 is inserted between the left fixed region 151a and the right fixed region 151b and the slit 415, and a part of the tube member 283 is drawn out and placed on the outer surface 410b side of the first band body 410, whereby the tube member 283 can be prevented from being pressed against the right hand H1 of the patient by the first band body 410 placed in the interdigital space fb.

In addition, in the hemostatic device 10, the inflatable member 210 has a sheet material connected to the cover member 100 in such a manner that an inner cavity 210a into which a fluid can be injected is defined with the lower surface region 132, and the edge 212 located on the lower end portion 302 side of the support member 300 in the sheet material is connected to the curved region 133.

In the hemostatic device 10, the edge 212 located at the lower end portion 302 of the support member 300 in the sheet material constituting the inflatable member 210 is connected to the curved region 133 formed by the cover member 100. Therefore, the inflatable member 210 is placed such that the center c1 of the inflatable member 210 is located at a position shifted to the proximal portion 122 side of the main body 110 in the longitudinal width direction of the main body 110. In the hemostatic device 10, the second band body 420 and the third band body 430 having a function of mainly applying a securing force for securing the inflatable member 210 to the right hand H1 are positioned on the proximal portion side (curved region 133 side) of the main body 110 of the cover member 100. Therefore, when the center c1 of the inflatable member 210 is displaced to the position on the proximal portion side of the main body 110 of the cover member 100, a securing force is easily applied from each of the band bodies 410 and 420 to the vicinity of the center c1 of the inflatable member 210 when the band bodies 410 and 420 are wrapped around the right hand H1. As a result, the distal portion side (first band body 410 side) of the inflatable member 210 is less likely to be lifted, and thus, the hemostatic device 10 can improve the compressive force applied to the first puncture site p1 by the inflatable member 210.

In addition, in the hemostatic device 10, the lower surface region 132 has a constricted portion 132a configured such that the width of the lower surface region 132 decreases from the first band body portion 410 side toward the curved region 133 side, and the constricted portion 132a is not fixed to the upper surface region 131.

In a state where the hemostatic device 10 is worn on the right hand H1, the deformation of the upper surface region 131 is not inhibited by the lower surface region 132 due to the constricted portion 132a located in the lower surface region 132, and the upper surface region 131 can be deformed along the support member 300. Therefore, in a state where the hemostatic device 10 is worn on the right hand H1, the proximal portion 122 of the cover member 100 of the hemostatic device 10 is deformed along both ends of the support member 300 in the width direction, so that a width of the proximal portion 122 of the cover member 100 can be reduced. Since the width of the proximal portion 122 of the cover member 100 decreases, the hemostatic device 10 can prevent an article or the like from being easily caught on the proximal portion 122 of the cover member 100. In particular, in the hemostatic device 10, the constricted portion 132a is not fixed to the upper surface region 131, whereby it is possible to prevent the cover member 100 from being hardly deformed in the vicinity of the constricted portion 132a. Accordingly, it is possible to effectively prevent the hemostatic device 10 from being bulky in a state where the hemostatic device 10 is worn on the right hand H1.

In the hemostatic device 10, the maximum width ws1 of the support member 300 is larger than the maximum width wh of the holding portion 130 between the curved region 133 and the ends 154a and 154b of the second fixed regions 152 on the curved region 133 side.

In the hemostatic device 10, the maximum width of the support member 300 (the width ws1 of the upper end portion 301) is larger than the maximum width wh of the holding portion 130, whereby it is possible to more reliably prevent the support member 300 from slipping out of the clearance between the fixed regions 150A and 150B and the curved region 133.

In addition, in the hemostatic device 10, the ends 154a and 154b, which are located on the curved region 133 side, of the fixed regions 150A and 150B are positioned on the first band body 410 side with respect to the intermediate portion 305 of the support member 300.

In the hemostatic device 10, the ends 154a and 154b, which are located on the curved region 133 side, of the fixed regions 150A and 150B are positioned on the first band body 410 side with respect to the intermediate portion 305 of the support member 300. Therefore, the hemostatic device 10 can ensure the mobility in the vicinity of the first ends 421 and 431 of the band bodies 420 and 430 when the band bodies 420 and 430 are wrapped around the right hand H1. Accordingly, it is possible to wrap the band bodies 420 and 430 of the hemostatic device 10 around the right hand H1 more easily and smoothly.

In the hemostatic device 10, the first band body 410 has a first end 411 located on the main body 110 side of the cover member 100, and the first end 411 of the first band body 410 is connected to the inner surface 110a side of the main body 110 in a state of being spaced apart from the first fixed region 151.

In the hemostatic device 10, the first end 411 of the first band body 410 is connected to the inner surface 110a side of the main body 110 of the cover member 100 in a state of being spaced apart from the first fixed region 151. The first band body 410 is spaced apart from the first fixed region 151. Therefore, when the first band body 410 of the cover member 100 is placed in the interdigital space fb, the first fixed region 151 and the first end 411 function as two-step joints. Therefore, the hemostatic device 10 can increase the mobility of the first band body 410 when the first band body 410 is placed in the interdigital space fb. In addition, since the first band body 410 is spaced apart from the first fixed region 151, the mobility in the lateral direction in the vicinity of the first end 411 can also be ensured. As a result, the first band body 410 of the hemostatic device 10 can be more easily wrapped around the interdigital space fb. The first band body 410 of the hemostatic device 10 can be deflected so as to follow the surface of the right hand H1, whereby it is possible to prevent the hemostatic device 10 from being bulky in a state of being worn on the right hand H1 of the patient. Therefore, it is possible to suitably prevent the hemostatic device 10 from coming into contact with another article or the like in a state of being worn on the right hand H1 of the patient.

### <Modification>

Next, modifications of the above-described embodiment will be described. In the description of the modifications, detailed descriptions of the members, the use procedure of the hemostatic device, and the like already described will be omitted, as appropriate. Furthermore, contents that are not particularly described in the modifications can be the same as those in the above-described embodiment.

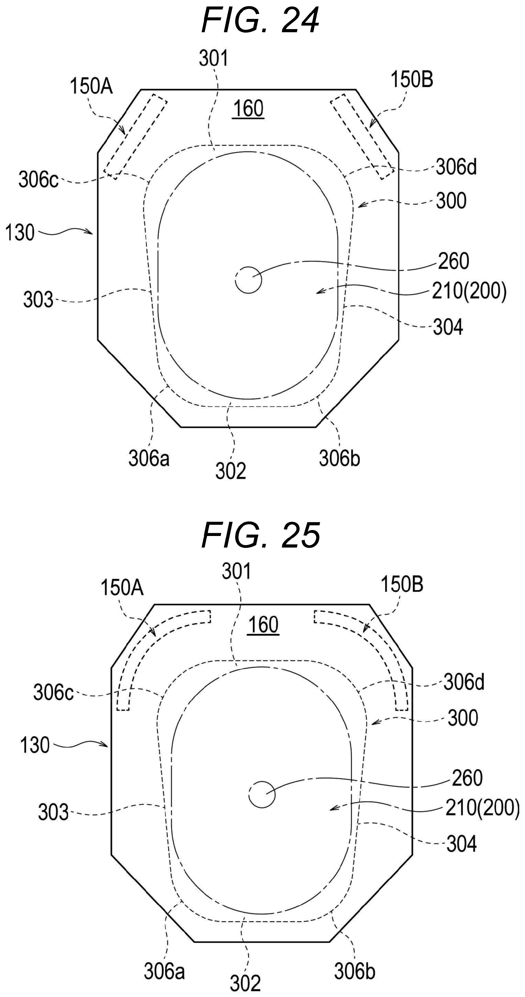

Figs. 24 and 25 illustrate modifications of the fixed regions 150A and 150B.

In the hemostatic device according to the present invention, the mode of the fixed regions 150A and 150B is not particularly limited as long as "the edge 121 of the lower surface region 132 has fixed regions 150A and 150B fixed to the upper surface region 131, and the fixed regions 150A and 150B are located on the distal side with respect to the support member 300 and are located at positions surrounding the edge portions 306c and 306d in plan view" as described in the above-described embodiment.

For example, as illustrated in Fig. 24, the fixed regions 150A and 150B can be configured to have a shape extending in an oblique direction so as to surround the edge portions 306c and 306d. Alternatively, as illustrated in Fig. 25, the fixed regions 150A and 150B can be configured to have, for example, a curved shape so as to surround the edge portions 306c and 306d. In addition, the fixed regions 150A and 150B illustrated in Figs. 24 and 25 and the fixed regions 150A and 150B described in the above-described embodiment can be freely combined.

While the hemostatic device according to the present invention has been described above by way of the embodiment and the modifications, the present invention is not limited only to the content described in the specification and can be appropriately changed on the basis of the description of the claims.

The shape, dimension, and the like of each portion of the hemostatic device are not particularly limited as long as hemostatic compression can be performed on the puncture site using the pressing member placed on the puncture site, and can be appropriately changed.

The specific shapes (planar shape and cross-sectional shape) of the inflatable member and the support member are not limited to the shapes illustrated in the embodiment.

The configuration of the pressing member that applies a compressive force to the puncture site is not limited to the inflatable member (balloon). The pressing member may include, for example, a resin material such as plastic configured to be able to apply a compressive force to the puncture site, a member made of gel or the like, an elastic material such as a sponge-like substance, an assembly of fibers such as cotton, metal, a member having a predetermined three-dimensional shape (spherical, ellipsoid, triangular pyramid, or the like), or a combination thereof as appropriate.

The hemostatic device can also be configured for the purpose of stopping bleeding from a site other than the puncture site formed in the hand. For example, the hemostatic device can also be applied to stop bleeding at a puncture site formed in an arm, a leg, or the like of a patient using the pressing member of the embodiment.

The present application is based on Japanese Patent Application No. 2023-137824 filed on August 28, 2023, the disclosure content of which is incorporated herein by reference in its entirety.

### Reference Signs List

10 Hemostatic device
100 Cover member
110 Main body of cover member
110a Inner surface of main body
110b Outer surface of main body
120 Sheet material
121 Edge of lower surface region
130 Holding portion
131 Upper surface region
132 Lower surface region
132a Constricted portion
133 Curved region
135 Insertion portion
150A Fixed region
150B Fixed region
151 First fixed region
151a Left fixed region
151b Right fixed region
152 Second fixed region
152a First part
152b First part
153a Second part
153b Second part
154a End of fixed region
154b End of fixed region
157 Clearance
160 Non-fixed region
200 Pressing member
210 Inflatable member
210a Inner cavity of inflatable member
212 Edge of inflatable member
260 Marker portion
281 Injection member
282 Buffer member
283 Tube member
300 Support member
300a Lower surface of support member
300b Upper surface of support member
301 Upper end portion of support member
302 Lower end portion of support member
303 Side surface portion of support member
304 Side surface portion of support member
305 Intermediate portion of support member
306a First edge portion
306b Second edge portion
306c Third edge portion
306d Fourth edge portion
410 First band body
410a Inner surface of first band body
410b Outer surface of first band body
411 First end of first band body
415 Slit
420 Second band body
425 Deflected portion
430 Third band body
435 Deflected portion
600 Introducer
C Center line of first band body
w1 Width of lower surface region
wg Width of clearance
ws1 Width of upper end portion of support member
ws2 Width of lower end portion of support member
t1 Thickness of upper end portion of support member
wh Maximum width of holding portion
H Hand
H1 Right hand
Ar Forearm
fb Interdigital space
Sb Snuff box
B1 Metacarpal bone of index finger
B2 Metacarpal bone of thumb
T1 Extensor pollicis longus muscle tendon
T2 Extensor pollicis brevis muscle tendon
V1 Blood vessel (distal radial artery)
V2 Artery located at snuff box
p1 First puncture site (puncture site)
p2 Second puncture site (puncture site)

## Claims

1. A hemostatic device comprising:
a cover member configured to cover a puncture site formed in a patient;
a pressing member disposed on the cover member and configured to compress the puncture site; and
a support member disposed on the cover member so as to cover the pressing member, wherein
the cover member includes a main body where the pressing member is located, a first band body configured to be placed between fingers of the patient and extending in a first direction from the main body, a second band body extending in a second direction different from the first direction from the main body, and a third band body facing the second band body across the pressing member and extending from the main body in a third direction different from the first direction and the second direction,
the main body has a two-layer structure including a holding portion that holds the support member,
the holding portion has an upper surface region located on an upper surface side of the support member, a lower surface region located on a lower surface side of the support member and facing the upper surface region across the support member, and a curved region connecting the upper surface region and the lower surface region on a proximal side of the support member,
the curved region is formed by folding back a part of the main body toward the first band body,
the support member includes an upper end portion located on a side of the first band body and serves as an end of the support member, a lower end portion serving as an end opposite to the upper end portion, an intermediate portion positioned between the upper end portion and the lower end portion, and an edge portion connecting the upper end portion and the intermediate portion,
an edge of the lower surface region has a fixed region fixed to the upper surface region, and
the fixed region is positioned on a distal side with respect to the support member and located at a position surrounding the edge portion in plan view.

2. The hemostatic device according to claim 1, wherein
the fixed region includes a first fixed region located on a distal side with respect to the upper end portion of the support member and a second fixed region provided continuously with the first fixed region and extending from the first fixed region toward the lower end portion of the support member,
the holding portion has a clearance between the support member and the first fixed region in plan view, and
a width of the clearance is equal to or larger than a thickness of the upper end portion of the support member.

3. The hemostatic device according to claim 2, wherein
the second band body and the third band body extend from the main body in the second direction and the third direction via the second fixed region located between the second and third band bodies and the main body, and
the second band body and the third band body have deflected portions located on a side of the first band body with respect to an end of the second fixed region located on a side of the curved region in plan view.

4. The hemostatic device according to claim 2, wherein
the pressing member is an inflatable member inflatable by injection of a fluid,
the inflatable member is connected to an injection member for injecting the fluid into the inflatable member via a tube member,
the first band body has a slit extending in a longitudinal direction of the first band body,
the first fixed region includes a left fixed region and a right fixed region facing the left fixed region across an extension of the slit, and
the tube member is located between the left fixed region and the right fixed region in plan view, passes through a non-fixed region in which the edge of the lower surface region is not fixed to the upper surface region, and is inserted from an inner surface side of the first band body toward an outer surface side of the first band body through the slit.

5. The hemostatic device according to claim 4, wherein
the inflatable member includes a sheet material connected to the cover member in such a manner that an inner cavity into which the fluid is capable of being injected is defined with the lower surface region, and
an edge located on a side of the lower end portion of the support member in the sheet material is connected to the curved region.

6. The hemostatic device according to claim 1, wherein
the lower surface region includes a constricted portion configured in such a manner that a width of the lower surface region decreases from a side of the first band body toward the curved region, and
the constricted portion is not fixed to the upper surface region.

7. The hemostatic device according to claim 2, wherein a maximum width of the support member is larger than a maximum width of the holding portion between the curved region and the end of the second fixed region on a side of the curved region.

8. The hemostatic device according to claim 1, wherein an end of the fixed region on a side of the curved region is located on a side of the first band body with respect to the intermediate portion of the support member.

9. The hemostatic device according to claim 2, wherein
the first band body has a first end located on a side of the main body of the cover member, and
the first end of the first band body is connected to an inner surface side of the main body in a state of being spaced apart from the first fixed region.
